# EUROPEAN PATENT APPLICATION

(11) **EP 4 497 753 A1**
(43) Date of publication of application: **29.01.2025**
(21) Application number: 24190698.1
(22) Date of filing: 24.07.2024
(51) Int. Cl.: C07H 17/065, A61Q 19/00, A61Q 19/08, C12P 19/18

(54) **CAMELLIA-DERIVED FLAVONOIDS**

(30) Priority: 27.07.2023 KR 20230098133; 28.06.2024 KR 20240085315
(71) Applicant: Amorepacific Corporation, Seoul 04386 (KR)
(72) Inventor: Rha, Chan Su, 17074 Yongin-si (KR); Ko, Jaeyoung, 17074 Yongin-si (KR); Cho, Si Young, 17074 Yongin-si (KR); Nam, Jin, 17074 Yongin-si (KR)
(74) Representative: Dr. Schön, Neymeyr & Partner Patentanwälte mbB

(57) **Abstract**

The present specification provides a novel kaempferol compound obtained by enzymatically treating kaempferol saccharides extracted from *Camellia oleifera* seed cake, which is discarded after oil extraction from camellia seeds, and a skin protection composition containing the same that is safe for the human body. The enzyme-treated-kaempferol compound has an increased molecular weight as sugar is additionally linked to the precursor kaempferol saccharide, and can exhibit a skin protection effect.

## Description

### [Technical Field]

Disclosed herein is a novel camellia-derived flavonoid, a kaempferol saccharide compound that is safe for the human body and has excellent skin protection effects, and a composition for protecting skin containing the same.

### [Background Art]

Plant-derived flavonoids have a C6-C3-C6 carbon skeleton structure with two phenyl groups mediated by a C3 chain, and they often exist in the form of saccharides combined with various saccharides. Plants produce specific glycoside structures for each individual through their unique biosynthetic pathway.

These flavonoid saccharides exhibit physiological activities such as antibacterial, anticancer, and anti-inflammatory, and the type and intensity of physiological activity varies depending on the position where the sugar is modified in the flavonoid structure, the sugar chain structure, and the molecular weight.

However, many existing plant-derived skin protection ingredients have the problem of using a large amount of plant resources or having low efficacy due to the specificity of molecular weight and molecular structure, making them of low utility value.

### [Disclosure]

### [Technical Problem]

In one aspect of the present disclosure, an object is to provide an enzyme-treated-kaempferol saccharide compound obtained from *Camellia oleifera* seed cake, a resource discarded after extraction of camellia seed oil, and a composition for protecting skin containing the same.

In another aspect of the present disclosure, an object is to provide an enzyme-treated kaempferol saccharide compound whose molecular size is adjusted by treating *Camellia oleifera* seed cake extract containing a large amount of kaempferol saccharide compound with an enzyme and a composition for protecting skin containing the same.

In another aspect of the present disclosure, an object is to provide a composition that contains an enzyme-treated-kaempferol saccharide compound as an active ingredient, is safe for the human body with few side effects, and has an excellent skin protection effect.

In another aspect of the present disclosure, an object is to provide a composition for protecting skin that inhibits the activity of 3-Phosphoinositide-dependent kinase 1 (PDK1), comprising an enzyme-treated-kaempferol saccharide compound as an active ingredient.

### [Technical Solution]

The present specification provides an enzyme-treated-kaempferol saccharide compound represented by any of the following Chemical Formulas 1 to 6, a stereoisomer thereof, a pharmaceutically acceptable salt thereof, a hydrate thereof, or a solvate thereof:

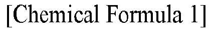

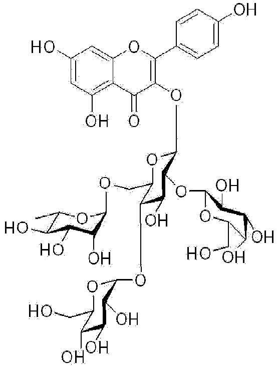

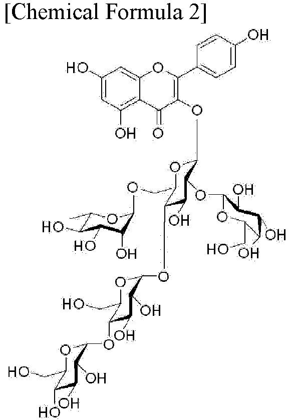

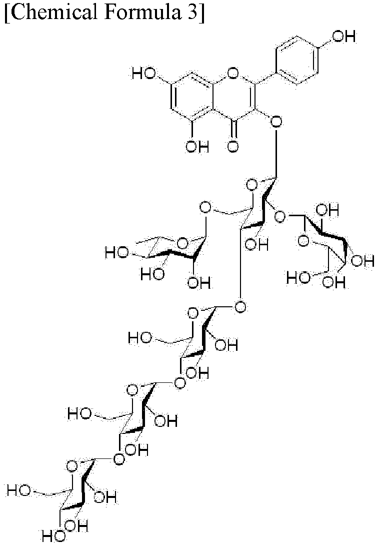

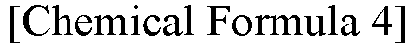

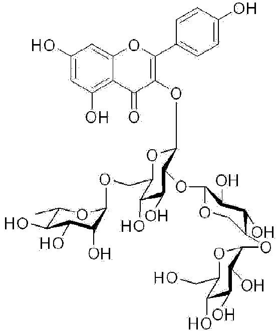

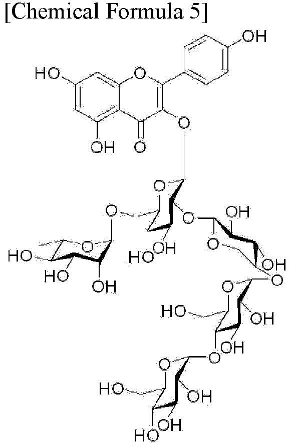

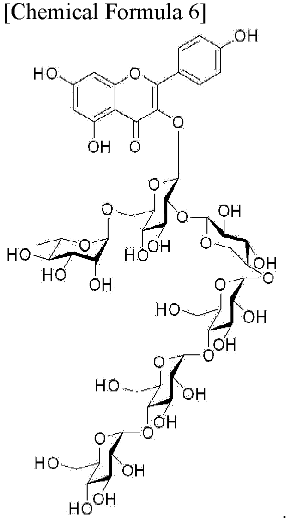

The present specification provides a composition for protecting skin, comprising the above-described enzyme-treated-kaempferol saccharide compound, the stereoisomer thereof, the pharmaceutically acceptable salt thereof, the hydrate thereof, or the solvate thereof as an active ingredient.

### [Advantageous Effects]

In one aspect, the enzyme-treated-kaempferol saccharide compound disclosed herein can inhibit the activity of 3-Phosphoinositide-dependent kinase 1 (PDK1).

In one aspect, the enzyme-treated-kaempferol saccharide compound disclosed herein can inhibit the activity of 3-Phosphoinositide-dependent kinase 1 (PDK1), thereby exhibiting a skin protection effect.

### [Brief Description of Drawings]

FIG. 1 is an ultraviolet absorption chromatogram at 365 nm for a *Camellia oleifera* seed cake extract and its enzymatic conversion product, an enzyme-treated-kaempferol compound, according to an example of the present disclosure.
FIG. 2 is an ultraviolet absorption chromatogram at 365 nm of the saccharide mixture obtained by processing Cameliaside A with an enzyme and its separated and purified product according to an example of the present disclosure.
FIG. 3 is an ultraviolet absorption chromatogram at 365 nm for the saccharide mixture obtained by processing Cameliaside B with an enzyme and its separated and purified product according to an example of the present disclosure.
FIG. 4 is an ultraviolet absorption chromatogram at 365 nm for the saccharide mixture obtained by processing Nicotiflorin with an enzyme and its separated and purified product according to an example of the present disclosure.
FIG. 5 is a graph showing the effect of inhibiting 3-Phosphoinositide-dependent kinase 1 (PDK1) activity on DMSO (blank), BX-795 (control), COSCE (*Camellia oleifera* seed cake extract), COSCE-P (purified product of *Camellia oleifera* seed cake extract), and COSCE-PeP (purified product of *Camellia oleifera* seed cake extract after enzyme treatment) according to an example of the present disclosure. The numerical unit above the bar is ug/mL.
FIG. 6 is a graph showing the effect of inhibiting 3-Phosphoinositide-dependent kinase 1 (PDK1) activity on DMSO (blank), BX-795 (control), CamA (Camelliaside A), CamB (Camelliaside B), and EM (enzymatically modified) according to an example of the present disclosure. The numerical unit above the bar is ug/mL. EM-CamA contains a compound of Chemical Formulas 1-3, and EM-CamB contains a compound of Chemical Formulas 4-6.
FIG. 7 is a graph showing the effect of inhibiting 3-Phosphoinositide-dependent kinase 1 (PDK1) activity on DMSO (blank), BX-795 (control), AG4 (Camelliaside A+one sugar), AG5 (Camelliaside A+two sugars), AG6 (Camelliaside A+three sugars) AG7 (Camelliaside A+four sugars), BG4 (Camelliaside B+one sugar), BG5 (Camelliaside B+two sugars), BG6 (Camelliaside B+three sugars), BG7 (Camelliaside B+four sugars), NG4 (Nicotiflorin+two sugars), NG5 (Nicotiflorin+three sugars), NG6 (Nicotiflorin+four sugars), NG7 (Nicotiflorin+five sugars) according to an example of the present disclosure.

### [Best Mode]

In present specification, when a part "comprises" a certain component, this means that it may further include other components rather than excluding other components unless specifically stated to the contrary.

Hereinafter, the present invention will be described in detail.

Exemplary embodiments of the present disclosure provide an enzyme-treated-kaempferol saccharide compound represented by any of the following Chemical Formulas 1 to 6, a stereoisomer thereof, a pharmaceutically acceptable salt thereof, a hydrate thereof, or a solvate thereof.

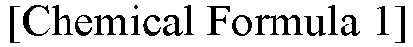

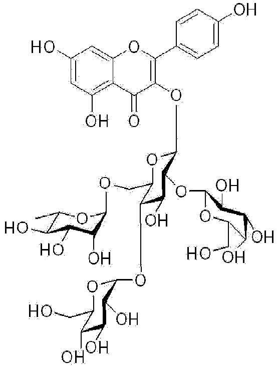

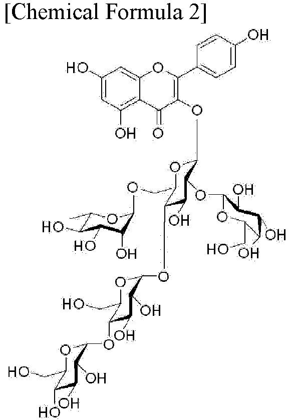

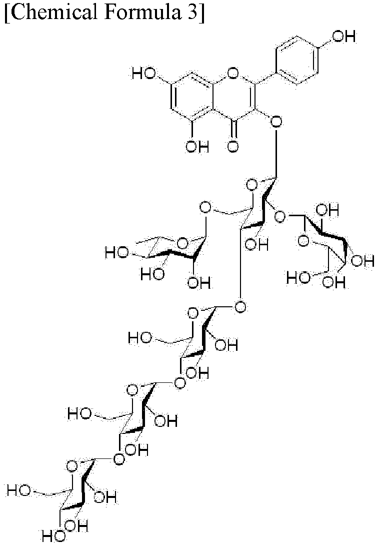

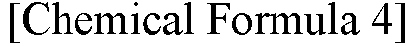

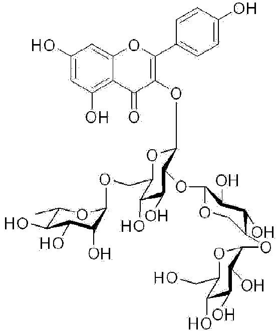

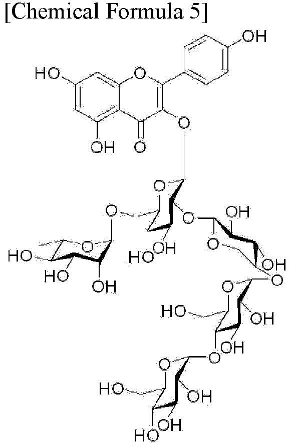

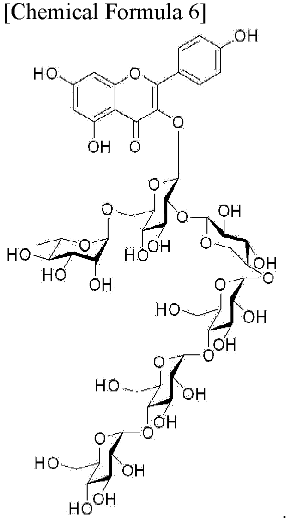

In another exemplary embodiments of the present disclosure, a method for protecting skin, comprising administering an effective amount of a composition comprising a kaempferol saccharide compound represented by any of the above Chemical Formulas 1 to 6, a stereoisomer thereof, a pharmaceutically acceptable salt thereof, a hydrate thereof, or a solvate thereof to a subject in need thereof is provided.

In another exemplary embodiments of the present disclosure, a use of a kaempferol saccharide compound represented by any of the above Chemical Formulas 1 to 6, a stereoisomer thereof, a pharmaceutically acceptable salt thereof, a hydrate thereof, or a solvate thereof for manufacturing a composition for protecting skin is provided.

In another exemplary embodiments of the present disclosure, a non-therapeutic use of a saccharide compound represented by any of the above Chemical Formulas 1 to 6, a stereoisomer thereof, a pharmaceutically acceptable salt thereof, a hydrate thereof, or a solvate thereof for protecting skin is provided.

As used herein, "isomers" specifically includes optical isomers (e.g., essentially pure enantiomers, essentially pure diastereomers, or mixtures thereof), as well as conformational isomers (i.e. isomers that differ only in the angle of one or more chemical bonds), position isomers (especially tautomers), or geometric isomers (e.g. cis-trans isomers).

As used herein, "essentially pure" means, for example, when used in relation to enantiomers or partial isomers, the specific compound, including enantiomers or partial isomers, is about 90% or more, preferably about 95% or more, more preferably about 97% or more or about 98% or more, even more preferably about 99% or more, and even more and more preferably about 99.5% or more (w/w).

As used herein, "pharmaceutically acceptable" means that a product can be approved or gets approved by a government or equivalent regulatory body for use in animals, more specifically in humans, by avoiding significant toxic effects when used in normal medicinal dosages, or is listed in a pharmacopoeia or otherwise recognized by a general pharmacopeia.

As used herein, "pharmaceutically acceptable salt" refers to a salt according to one aspect of the present disclosure that is pharmaceutically acceptable and has the desired pharmacological activity of the parent compound. The salts may comprise (1) formed with inorganic acids such as hydrochloric acid, hydrobromic acid, sulfuric acid, nitric acid, phosphoric acid, etc.; acid addition salts formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo [2,2,2]-oct-2-ene-1-carboxylic acid, glucoheptonic acid, 3-phenylpropionic acid, trimethylacetic acid, tert -butylacetic acid, lauryl sulfate, gluconic acid, glutamic acid, hydroxynaphthoic acid, salicylic acid, stearic acid, and muconic acid; or (2) a salt formed when an acidic proton present in the parent compound is replaced.

In this specification, "hydrate" refers to a compound to which water is bound, and is a broad concept that includes embedded compounds in which there is no chemical bond between water and the compound.

In this specification, "solvate" refers to a higher-order compound formed between a solute molecule or ion and a solvent molecule or ion.

Camelliaside A, a precursor of a compound represented by any one of Chemical Formulas 1 to 3 according to an embodiment of the present disclosure, may be represented by the following Chemical Formula A, and the results of nuclear magnetic resonance analysis of the kaempferol saccharide compound represented by Chemical Formula A are shown in Table 1 below.

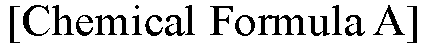

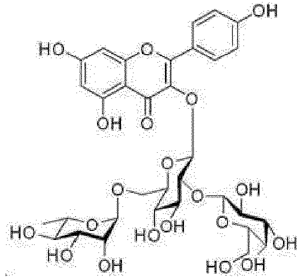

**[Table 1]**

| **¹H- and ¹³C-NMR data of camelliaside A in D₂O** | | |
|---|---|---|
| | ¹H-NMR | ¹³C-NMR |
| 2 | | 160.83 |
| 3 | | 136.14 |
| 4 | | 180.67 |
| 5 | | 159.33 |
| 6 | 6.10 (s) | 101.95 |
| 7 | | 166.57 |
| 8 | 6.21 (br s) | 97.55 |
| 9 | | 162.77 |
| 10 | | 107.11 |
| 1' | | 124.34 |
| 2' | 7.81 (d, *J*=8.0 Hz) | 134.00 |
| 3' | 6.83 (d, *J*=8.0 Hz) | 118.02 |
| 4' | | 161.47 |
| 5' | 6.83 (d, *J*=8.0 Hz) | 118.02 |
| 6' | 7 81 (d, *J*=8.0 Hz) | 134.00 |
| 3Glc-1" | 5.06 (d, *J*=7.2 Hz) | 102.68 |
| 3Glc-2" | 3.91 (dd, *J*=7.2, 84 Hz) | 81.95 |
| 3Glc-3" | 3.68 (dd, *J*=9.0, 84 Hz) | 78.75 |
| 3Glc-4" | 340 (dd, *J*=9.0, 8.4 Hz) | 72.47 |
| 3Glc-5" | 358 (brdd, *J*=8.4, 6.0 Hz) | 77.48 |
| 3Glc-6" | 3.37 (dd, *J*=11.4, 6.0 Hz) | 70.06 |
| | 3.77 (br d, *J*=11.4 Hz) | |
| 6"Rha-1‴ | 454 (s) | 103.47 |
| 6"Rha-2‴ | 3.67 (br s) | 73.00 |
| 6"Rha-3‴ | 3.40 (br d, *J*=8.4 Hz) | 72.40 |
| 6"Rha-4"' | 3.29 (dd, *J*=9.0, 8.4 Hz) | 74.64 |
| 6"Rha-5‴ | 3.39 (qd, *J*=6.0, 9.0 Hz) | 71.46 |
| 6"Rha-6‴ | 1.07 (d, *J*=6.0 Hz) | 19.32 |
| 2"Gal-1ʺʺ | 492 (d, *J*=84 Hz) | 105.04 |
| 2"Gal-2"" | 346 (dd, *J*=8.4, 9.0 Hz) | 76.40 |
| 2"Gal-3"" | 358 (dd, *J*=9.0, 3 6 Hz) | 78.57 |
| 2"Gal-4ʺʺ | 349 (m) | 72 82 |
| 2"Gal-5"" | 3.48 (m) | 78.97 |
| 2"Gal-6"" | 380 (dd, *J*=11.4, 54 Hz) | 63.76 |
| | 3.92 (dd, *J*=11.4, 6.6 Hz) | |

The results of nuclear magnetic resonance analysis of the enzyme-treated-kaempferol saccharide compound represented by the above Chemical Formula 1 are shown in Table 2.

**[Table 2]**

| **¹H- and ¹³C-NMR data of camelliaside A-G4 in D₂O** | | |
|---|---|---|
| | ¹H-NMR | ¹³C-NMR |
| 2 | | 160.69 |
| 3 | | 136.03 |
| 4 | | 180.55 |
| 5 | | 159.11 |
| 6 | 6.09 (s) | 101.58 |
| 7 | | 165.60 |
| 8 | 6.22 (br s) | 97.25 |
| 9 | | 162.68 |
| 10 | | 107.18 |
| 1' | | 124.23 |
| 2' | 7.79 (br.s) | 133.87 |
| 3' | 6.83 (br.s) | 117.89 |
| 4' | | 161.29 |
| 5' | 6.83 (br.s) | 117.89 |
| 6' | 7.79 (br.s) | 133.87 |
| 3Glc-1" | 5.08 (d, *J*=6.6 Hz) | 102.45 |
| 3Glc-2" | 3.88 (dd, *J*=6.6, 8.4 Hz) | 81.88 |
| 3Glc-3" | 3.82 (dd, *J*=9.0, 8.4 Hz) | 78.54 |
| 3Glc-4" | 3.67 (dd, *J*=9.0, 8.4 Hz) | 79.68 |
| 3Glc-5" | 3.66 (brdd, *J*=8.4, 6.0 Hz) | 78.79 |
| 3Glc-6" | 3.35 (dd, *J*=11.4, 6.0 Hz) | 69.85 |
| | 3.75 (br d, *J*=11.4 Hz) | |
| 6"Rha-1‴ | 4.52 (s) | 103.29 |
| 6"Rha-2‴ | 3.66 (br s) | 72.67 |
| 6"Rha-3‴ | 3.40 (br d, *J*=8.4 Hz) | 72.24 |
| 6"Rha-4‴ | 3.26 (dd, *J*=9.0, 8.4 Hz) | 74.50 |
| 6"Rha-5‴ | 3.38 (qd, *J*=6.0, 9.0 Hz) | 71.30 |
| 6"Rha-6‴ | 1.04 (d, *J*=6.0 Hz) | 19.32 |
| 2"Gal-1ʺʺ | 4.89 (d, *J*=7.8 Hz) | 104.75 |
| 2"Gal-2ʺʺ | 3.46 (dd, *J*=8.4, 9.0 Hz) | 76.10 |
| 2"Gal-3"" | 3.56 (dd, *J*=9.0, 9.0 Hz) | 77.49 |
| 2"Gal-4ʺʺ | 3.46 (m) | 72.85 |
| 2"Gal-5ʺʺ | 3.41 (m) | 77.37 |
| 2"Gal-6ʺʺ | 3.80 (m) | 63.54 |
| | 3.88 (m) | |
| 4"Glc-1‴ʺ | 5.40 (d, *J*=3.6 Hz) | 102.49 |
| 4"Glc-2‴ʺ | 3.59 (dd, *J*=9.6, 3.6 Hz) | 74.46 |
| 4"Glc-3‴ʺ | 3.71 (dd, *J*=9.0, 9.0 Hz) | 75.42 |
| 4"Glc-4‴ʺ | 3.42 (m) | 72.03 |
| 4"Glc-5‴ʺ | 3.73 (m) | 75.61 |
| 4"Glc-6‴ʺ | 3.78 (m) | 63.21 |
| | 3.85 (m) | |

The results of nuclear magnetic resonance analysis of the kaempferol saccharide compound represented by the above Chemical Formula 2 are shown in Table 3.

**[Table 3]**

| **¹H- and ¹³C-NMR data of camelliaside A-G5 in D₂O** | | |
|---|---|---|
| | ¹H-NMR | ¹³C-NMR |
| 2 | | 160.85 |
| 3 | | 136.18 |
| 4 | | 180.76 |
| 5 | | 159.37 |
| 6 | 6.15 (s) | 101.82 |
| 7 | | 165.95 |
| 8 | 6.27 (br s) | 97.48 |
| 9 | | 162.88 |
| 10 | | 107.38 |
| 1' | | 124.41 |
| 2' | 7.84 (br.s) | 134.03 |
| 3' | 6.87 (br.s) | 118.08 |
| 4' | | 161.49 |
| 5' | 6.87 (br.s) | 118.08 |
| 6' | 7.84 (br.s) | 134.03 |
| 3Glc-1" | 5.13 (d, *J*=6.0 Hz) | 102.59 |
| 3Glc-2" | 3.90 (dd, *J*=6.0, 8.4 Hz) | 82.12 |
| 3Glc-3" | 3.82 (dd, *J*=9.0, 8.4 Hz) | 78.69 |
| 3Glc-4" | 3.68 (dd, *J*=9.0, 8.4 Hz) | 80.11 |
| 3Glc-5" | 3.66 (brdd, *J*=8.4, 6.0 Hz) | 78.90 |
| 3Glc-6" | 3.35 (dd, *J*=11.4, 6.0 Hz) | 70.06 |
| | 3.75 (br d, *J*=11.4 Hz) | |
| 6"Rha-1‴ | 4.55 (s) | 103.46 |
| 6"Rha-2‴ | 3.66 (br s) | 72.84 |
| 6"Rha-3‴ | 3.40 (br d, *J*=8.4 Hz) | 72.43 |
| 6"Rha-4‴ | 3.28 (dd, *J*=9.0, 8.4 Hz) | 74.65 |
| 6"Rha-5‴ | 3.38 (qd, *J*=6.0, 9.0 Hz) | 71.47 |
| 6"Rha-6‴ | 1.06 (d, *J*=6.0 Hz) | 19.33 |
| 2"Gal-1"" | 4.95 (d, *J*=7.8 Hz) | 104.97 |
| 2"Gal-2ʺʺ | 3.48 (dd, *J*=8.4, 9.0 Hz) | 76.22 |
| 2"Gal-3"" | 3.56 (dd, *J*=9.0, 9.0 Hz) | 77.63 |
| 2"Gal-4ʺʺ | 3.46 (m) | 73.01 |
| 2"Gal-5ʺʺ | 3.45 (m) | 77.56 |
| 2"Gal-6ʺʺ | 3.80 (m) | 63.64 |
| | 3.88 (m) | |
| 4"Glc-1‴ʺ | 5.40 (d, *J*=3.6 Hz) | 102.72 |
| 4"Glc-2‴ʺ^{.} | 3.65 (dd, *J*=9.6, 3.6 Hz) | 74.43 |
| 4"Glc-3‴ʺ | 3.99 (dd, *J*=9.6, 9.6 Hz) | 76.22 |
| 4"Glc-4‴ʺ | 3.68 (m) | 79.72 |
| 4"Glc-5‴ʺ | 3.87 (m) | 74.08 |
| 4"Glc-6‴ʺ | 3.77 (m) | 63.32 |
| | 3.84 (m) | |
| 4‴ʺGlc-1‴‴ | 5.40 (d, *J*=3.6 Hz) | 102.59 |
| 4‴ʺGlc-2‴‴ | 3.60 (dd, *J*=9.6, 3.6 Hz) | 74.65 |
| 4‴ʺGlc-3‴‴ | 3.71 (dd, *J*=9.0, 3.6 Hz) | 75.57 |
| 4‴ʺGlc-4‴‴ | 3.38 (m) | 72.15 |
| 4‴ʺGlc-5‴‴ | 3.73 (m) | 75.76 |
| 4‴ʺGlc-6‴‴ | 3.78 (m) | 63.32 |
| | 3.85 (m) | |

The results of nuclear magnetic resonance analysis of the kaempferol saccharide compound represented by the above Chemical Formula 3 are shown in Table 4.

**[Table 4]**

| **¹H**- **and ¹³C-NMR data of camelliaside A-G6 in D₂O** | | |
|---|---|---|
| | ¹H-NMR | ¹³C-NMR |
| 2 | | 160.93 |
| 3 | | 136.18 |
| 4 | | 180.82 |
| 5 | | 159.43 |
| 6 | 6.18 (s) | 101.86 |
| 7 | | 165.90 |
| 8 | 6.32 (br s) | 98.94 |
| 9 | | 162.93 |
| 10 | | 107.45 |
| 1' | | 124.41 |
| 2' | 7.86 (br.s) | 134.03 |
| 3' | 6.89 (br.s) | 118.08 |
| 4' | | 161.51 |
| 5' | 6.89 (br.s) | 118.08 |
| 6' | 7.86 (br.s) | 134.03 |
| 3Glc-1" | 5.13 (d, *J*=6.0 Hz) | 102.71 |
| 3Glc-2" | 3.90 (dd, *J*=6.0, 8.4 Hz) | 82.12 |
| 3Glc-3" | 3.82 (dd, *J*=9.0, 8.4 Hz) | 78.69 |
| 3Glc-4" | 3.68 (dd, *J*=9.0, 8.4 Hz) | 80.11 |
| 3Glc-5" | 3.66 (brdd, *J*=8.4, 6.0 Hz) | 78.90 |
| 3Glc-6" | 3.35 (dd, *J*=11.4, 6.0 Hz) | 70.06 |
| | 3.75 (br d, *J*=11.4 Hz) | |
| 6"Rha-1‴ | 4.55 (s) | 103.47 |
| 6"Rha-2‴ | 3.66 (br s) | 72.84 |
| 6"Rha-3‴ | 3.40 (br d, *J*=8.4 Hz) | 72.43 |
| 6"Rha-4‴ | 3.28 (dd, *J*=9.0, 8.4 Hz) | 74.65 |
| 6"Rha-5‴ | 3.38 (qd, *J*=6.0, 9.0 Hz) | 71.47 |
| 6"Rha-6‴ | 1.06 (d, *J*=4.8 Hz) | 19.33 |
| 2"Gal-1"" | 4.90 (d, *J*=7.8 Hz) | 105.02 |
| 2"Gal-2ʺʺ | 3.48 (dd, *J*=8.4, 9.0 Hz) | 76.23 |
| 2"Gal-3ʺʺ | 3.56 (dd, *J*=9.0, 9.0 Hz) | 77.64 |
| 2"Gal-4ʺʺ | 3.46 (m) | 73.01 |
| 2"Gal-5"" | 3.45 (m) | 77.59 |
| 2"Gal-6ʺʺ | 3.80 (m) | 63.64 |
| | 3.88 (m) | |
| 4"Glc-1‴ʺ | 5.40 (d, *J*=3.6 Hz) | 102.64 |
| 4"Glc-2‴ʺ | 3.65 (dd, *J*=9.6, 3.6 Hz) | 74.42 |
| 4"Glc-3‴ʺ | 3.97 (dd, *J*=9.6, 9.6 Hz) | 76.19 |
| 4"Glc-4‴ʺ | 3.67 (m) | 79.72 |
| 4"Glc-5‴ʺ' | 3.88 (m) | 74.07 |
| 4"Glc-6‴ʺ | 3.77 (m) | 63.33 |
| | 3.84 (m) | |
| 4‴ʺGlc-1‴‴ | 5.39 (d, *J*=3.6 Hz) | 102.71 |
| 4‴ʺGlc-2‴‴ | 3.65 (dd, *J*=9.6, 3.6 Hz) | 74.49 |
| 4‴ʺGlc-3‴‴ | 3.98 (dd, *J*=9.6, 9.6 Hz) | 76.22 |
| 4‴ʺGlc-4*‴‴* | 3.68 (m) | 79.72 |
| 4‴ʺGlc-5‴‴ | 3.87 (m) | 74.09 |
| 4‴ʺGlc-6‴‴ | 3.77 (m) | 63.29 |
| | 3.84 (m) | |
| 4‴‴Glc-1‴ʺʺ | 5.39 (d, *J*=3.6 Hz) | 102.58 |
| 4‴‴Glc-2‴ʺʺ | 3.60 (dd, *J*=9.6, 3.6 Hz) | 74.65 |
| 4‴‴Glc-3‴ʺʺ | 3.71 (dd, *J*=9.0, 3.6 Hz) | 75.58 |
| 4‴‴Glc-4‴ʺʺ | 3.38 (m) | 72.17 |
| 4‴ʺGlc-5‴ʺʺ | 3.73 (m) | 75.77 |
| 4‴ʺGlc-6‴ʺʺ | 3.78 (m) | 63.24 |
| | 3.85 (m) | |

The kaempferol heptasaccharide compound obtained by adding four sugars to the above-described Camelliaside A as a precursor may be represented by the following Chemical Formula A-1, and the results of nuclear magnetic resonance analysis of the compound of Chemical Formula A-1 are shown in the below Table 5.

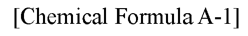

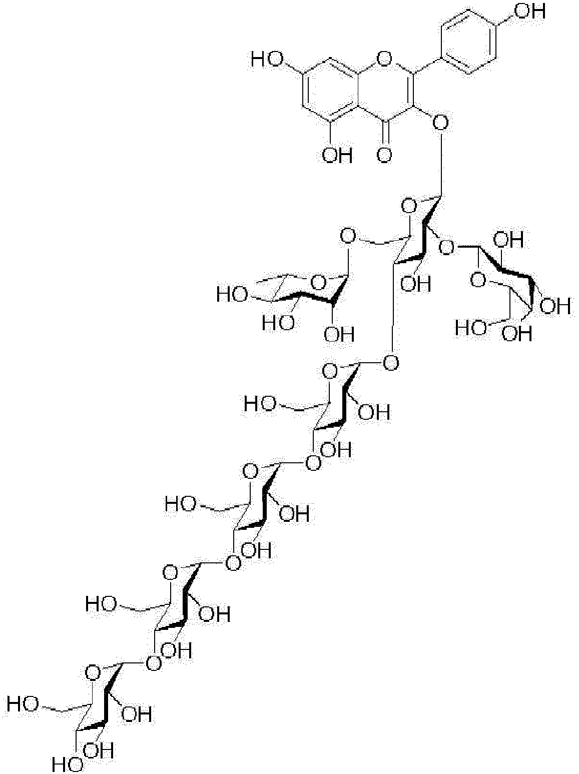

**[Table 5]**

| **¹H- and ¹³C-NMR data of camelliaside A-G7 in D₂O** | | |
|---|---|---|
| | ¹H-NMR | ¹³C-NMR |
| 2 | | 160.84 |
| 3 | | 136.12 |
| 4 | | 180.82 |
| 5 | | 159.52 |
| 6 | 6.24 (s) | 101.86 |
| 7 | | 166.33 |
| 8 | 6.35 (br s) | 97.71 |
| 9 | | 162.92 |
| 10 | | 107.40 |
| 1' | | 124.48 |
| 2' | 7.89 (br.s) | 134.06 |
| 3' | 6.91 (br.s) | 118.13 |
| 4' | | 161.55 |
| 5' | 6.91 (br.s) | 118.13 |
| 6' | 7.89 (br.s) | 134.06 |
| 3Glc-1" | 5.19 (d, *J*=6.0 Hz) | 102.50 |
| 3Glc-2" | 3.90 (dd, *J=*6.0, 8.4 Hz) | 82.32 |
| 3Glc-3" | 3.82 (dd, *J*=9.0, 8.4 Hz) | 78.64 |
| 3Glc-4" | 3.68 (dd, *J*=9.0, 8.4 Hz) | 79.91 |
| 3Glc-5" | 3.66 (brdd, *J*=8.4, 6.0 Hz) | 78.91 |
| 3Glc-6" | 3.35 (dd, *J*=11.4, 6.0 Hz) | 70.07 |
| | 3.75 (br d, *J*=11.4 Hz) | |
| 6"Rha-1‴ | 4.56 (s) | 103.45 |
| 6"Rha-2‴ | 3.66 (br s) | 72.84 |
| 6"Rha-3‴ | 3.40 (br d, *J*=8.4 Hz) | 72.48 |
| 6"Rha-4‴ | 3.28 (dd, *J*=9.0, 8.4 Hz) | 74.65 |
| 6"Rha-5‴ | 3.38 (qd, *J*=6.0, 9.0 Hz) | 71.49 |
| 6"Rha-6‴ | 1.06 (d, *J*=4.8 Hz) | 19.30 |
| 2"Gal-1ʺʺ | 4.88 (d, *J*=7.8 Hz) | 105.04 |
| 2"Gal-2ʺʺ | 3.48 (dd, *J*=8.4, 9.0 Hz) | 76.20 |
| 2"Gal-3"" | 3.56 (dd, *J*=9.0, 9.0 Hz) | 77.62 |
| 2"Gal-4ʺʺ | 3.46 (m) | 72.99 |
| 2"Gal-5"" | 3.45 (m) | 77.62 |
| 2"Gal-6ʺʺ | 3.80 (m) | 63.56 |
| | 3.88 (m) | |
| 4"Glc-1‴ʺ | 5.39 (d, *J*=3.0 Hz) | 102.61 |
| 4"Glc-2‴ʺ | 3.65 (dd, *J*=9.6, 3.6 Hz) | 74.41 |
| 4"Glc-3‴ʺ | 3.97 (dd, *J*=9.6, 9.6 Hz) | 76.20 |
| 4"Glc-4‴ʺ | 3.67 (m) | 79.74 |
| 4"Glc-5‴ʺ | 3.88 (m) | 74.06 |
| 4"Glc-6‴ʺ | 3.77 (m) | 63.31 |
| | 3.84 (m) | |
| 4‴ʺGlc-1‴‴ | 5.39 (d, *J*=3.6 Hz) | 102.72 |
| 4‴ʺGlc-2‴‴ | 3.65 (dd, *J*=9.6, 3.6 Hz) | 74.45 |
| 4‴ʺGlc-3‴‴ | 3.98 (dd, *J*=9.6, 9.6 Hz) | 76.22 |
| 4‴ʺGlc-4*‴‴* | 3.68 (m) | 80.12 |
| 4‴ʺGlc-5‴‴ | 3.87 (m) | 74.06 |
| 4‴ʺGlc-6‴‴ | 3.77 (m) | 63.33 |
| | 3.84 (m) | |
| 4‴‴Glc-1‴ʺʺ | 5.39 (d, *J*=3.6 Hz) | 102.60 |
| 4‴‴Glc-2‴ʺʺ | 3.65 (dd, *J*=9.6, 3.6 Hz) | 74.41 |
| 4‴‴Glc-3‴ʺʺ | 3.98 (dd, *J*=9.6, 9.6 Hz) | 76.20 |
| 4‴‴Glc-4‴ʺʺ | 3.68 (m) | 79.91 |
| 4‴‴Glc-5‴ʺʺ | 3.87 (m) | 74.06 |
| 4‴‴Glc-6‴ʺʺ | 3.77 (m) | 63.31 |
| | 3.84 (m) | |
| 4‴ʺʺGlc-1‴‴ʺ | 5.39 (d, *J*=3.6 Hz) | 102.60 |
| 4‴ʺʺGlc-2‴‴ʺ | 3.60 (dd, *J*=9.6, 3.6 Hz) | 74.63 |
| 4‴ʺʺGlc-3‴‴ʺ | 3.71 (dd, *J*=9.0, 3.6 Hz) | 75.56 |
| 4‴ʺʺGlc-4‴‴ʺ | 3.38 (m) | 72.14 |
| 4‴ʺʺGlc-5‴‴ʺ | 3.73 (m) | 75.75 |
| 4‴ʺʺGlc-6‴‴ʺ | 3.78 (m) | 63.22 |
| | 3.85 (m) | |

Camelliaside B, a precursor of a compound represented by any one of Chemical Formulas 4 to 6 according to an embodiment of the present disclosure, may be represented by the following Chemical Formula B, and the results of nuclear magnetic resonance analysis of the kaempferol saccharide compound represented by Chemical Formula B are shown in Table 6.

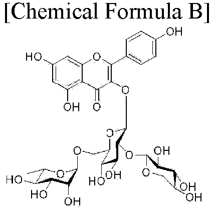

**[Table 6]**

| **¹H**- **and ¹³C-NMR data of camelliaside B in D₂O** | | |
|---|---|---|
| | ¹H-NMR | ¹³C-NMR |
| 2 | | 160.28 |
| 3 | | 136.19 |
| 4 | | 180.67 |
| 5 | | 159.23 |
| 6 | 6.10 (s) | 101.86 |
| 7 | | 166.42 |
| 8 | 6.16 (br s) | 97.50 |
| 9 | | 162.76 |
| 10 | | 107.11 |
| 1' | | 124.19 |
| 2' | 774 (d, *J*=7.8 Hz) | 133.90 |
| 3' | 676 (d, *J*=7.8 Hz) | 117.89 |
| 4' | | 161.39 |
| 5' | 6.76 (d, *J*=7.8 Hz) | 117.89 |
| 6' | 7.74 (d, *J*=7.8 Hz) | 133.90 |
| 3Glc-1" | 5.04 (d, *J*=7.2 Hz) | 102.49 |
| 3Glc-2" | 3.78 (dd, *J*=7.2, 9.0 Hz) | 82.59 |
| 3Glc-3" | 3.54 (dd, *J*=9.0, 9.0 Hz) | 78.36 |
| 3Glc-4" | 3.40 (dd, *J*=9.0, 84 Hz) | 72.47 |
| 3Glc-5" | 3.58 (brdd, *J*=8.4, 6.0 Hz) | 78.87 |
| 3Glc-6" | 3.41 (dd, *J*=11.4, 6.0 Hz) | 69.90 |
| | 3.77 (br d, *J*=11.4 Hz) | |
| 6"Rha-1‴ | 4.55 (s) | 103.44 |
| 6"Rha-2‴ | 3.66 (br s) | 72.82 |
| 6"Rha-3‴ | 3.49 (dd, *J*=8.4, 3.0 Hz) | 72.98 |
| 6"Rha-4‴ | 3.29 (dd, *J*=9.0, 8.4 Hz) | 74.64 |
| 6"Rha-5‴ | 3.39 (qd, *J*=6.0, 9.0 Hz) | 71.45 |
| 6"Rha-6‴ | 1.07 (d, *J*=6.0 Hz) | 19.31 |
| 2"Xyl-1ʺʺ | 4.83 (d, *J*=8.4 Hz) | 106.23 |
| 2"Xyl-2ʺʺ | 3.46 (dd, *J*=8.4, 9.0 Hz) | 76.25 |
| 2"Xyl-3ʺʺ | 3.40 (dd, *J*=9.0, 9.0 Hz) | 77.54 |
| 2"Xyl-4ʺʺ | 3.70 (ddd, *J*=9.0, 9.6, 5.4 Hz) | 72.35 |
| 2"Xyl-5ʺʺ | 3.37 (dd, *J*=12.0, 9.6 Hz) | 68.07 |
| | 4.05 (dd, *J*=12.0, 5.4 Hz) | |

The results of nuclear magnetic resonance analysis of the kaempferol saccharide compound represented by the above Chemical Formula 4 are shown in Table 7.

**[Table 7]**

| **¹H- and ¹³C-NMR data of camelliaside B-G4 in D₂O** | | |
|---|---|---|
| | ¹H-NMR | ¹³C-NMR |
| 2 | | 160.28 |
| 3 | | 136.19 |
| 4 | | 180.67 |
| 5 | | 159.23 |
| 6 | 6.09 (s) | 101.86 |
| 7 | | 166.42 |
| 8 | 6.14 (br s) | 97.50 |
| 9 | | 162.76 |
| 10 | | 107.11 |
| 1' | | 124.19 |
| 2' | 7.74 (d, *J*=7.8 Hz) | 133.90 |
| 3' | 6.78 (d, *J*=7.8 Hz) | 117.89 |
| 4' | | 161.39 |
| 5' | 6.78 (d, *J*=7.8 Hz) | 117.89 |
| 6' | 7.74 (d, *J*=7.8 Hz) | 133.90 |
| 3Glc-1" | 5.13 (d, *J*=7.2 Hz) | 102.49 |
| 3Glc-2" | 3.81 (dd, *J*=6.6, 8.4 Hz) | 82.45 |
| 3Glc-3" | 3.45 (dd, *J*=9.0, 8.4 Hz) | 77.71 |
| 3Glc-4" | 3.46 (dd, *J*=9.0, 8.4 Hz) | 72.43 |
| 3Glc-5" | 3.69 (brdd, *J*=8.4, 6.0 Hz) | 79.06 |
| 3Glc-6" | 3.35 (dd, *J*=11.4, 6.0 Hz) | 69.91 |
| | 3.75 (br d, *J*=11.4 Hz) | |
| 6"Rha-1‴ | 4.59 (s) | 103.44 |
| 6"Rha-2‴ | 3.66 (br s) | 72.93 |
| 6"Rha-3‴ | 3.54 (dd, *J*=8.4, 3.0 Hz) | 73.11 |
| 6"Rha-4‴ | 3.31 (dd, *J*=9.0, 8.4 Hz) | 74.79 |
| 6"Rha-5‴ | 3.45 (qd, *J*=6.0, 9.0 Hz) | 71.54 |
| 6"Rha-6‴ | 1.09 (d, *J*=6.0 Hz) | 19.45 |
| 2"Xyl-1ʺʺ | 4.85 (d, *J*=7.2 Hz) | 106.23 |
| 2"Xyl-2ʺʺ | 3.46 (dd, *J*=7.2, 9.0 Hz) | 77.03 |
| 2"Xyl-3ʺʺ | 3.68 (dd, *J*=9.0, 9.0 Hz) | 75.20 |
| 2"Xyl-4ʺʺ | 3.70 (ddd, *J*=9.0, 9.6, 5.4 Hz) | 81.19 |
| 2"Xyl-5ʺʺ | 3.37 (dd, *J*=12.0, 9.6 Hz) | 67.01 |
| | 4.05 (dd, *J*=12.0, 5.4 Hz) | |
| 4ʺʺGlc-1‴ʺ | 5.20 (d, *J*=3.6 Hz) | 102.49 |
| 4ʺʺGlc-2‴ʺ | 3.59 (dd, *J*=9.6, 3.6 Hz) | 74.54 |
| 4ʺʺGlc-3‴ʺ | 3.71 (dd, *J*=9.0, 9.0 Hz) | 75.76 |
| 4ʺʺGlc-4‴ʺ | 3.45 (m) | 72.25 |
| 4ʺʺGlc-5‴ʺ | 3.49 (m) | 75.94 |
| 4ʺʺGlc-6‴ʺ | 3.80 (m) | 63.31 |
| | 3.81 (m) | |

The results of nuclear magnetic resonance analysis of the kaempferol saccharide compound represented by the above Chemical Formula 5 are shown in Table 8.

**[Table 8]**

| **¹H- and ¹³C-NMR data of camelliaside B-G5 in D₂O** | | |
|---|---|---|
| | ¹H-NMR | ¹³C-NMR |
| 2 | | 160.03 |
| 3 | | 136.13 |
| 4 | | 180.66 |
| 5 | | 159.13 |
| 6 | 6.11 (s) | 101.68 |
| 7 | | 165.69 |
| 8 | 6.19 (br s) | 97.33 |
| 9 | | 162.86 |
| 10 | | 107.36 |
| 1' | | 124.28 |
| 2' | 7.77 (br.s) | 133.88 |
| 3' | 6.79 (br.s) | 117.93 |
| 4' | | 161.40 |
| 5' | 6.79 (br.s) | 117.93 |
| 6' | 7.77 (br.s) | 133.88 |
| 3Glc-1" | 5.19 (d, *J*=6.0 Hz) | 102.66 |
| 3Glc-2" | 3.90 (dd, *J*=6.0, 8.4 Hz) | 82.62 |
| 3Glc-3" | 3.45 (dd, *J*=9.0, 8.4 Hz) | 77.71 |
| 3Glc-4" | 3.43 (dd, *J*=9.0, 8.4 Hz) | 72.39 |
| 3Glc-5" | 3.67 (brdd, *J*=8.4, 6.0 Hz) | 78.92 |
| 3Glc-6" | 3.46 (dd, *J*=11.4, 6.0 Hz) | 69.84 |
| | 3.80 (br d, *J*=11.4 Hz) | |
| 6"Rha-1‴ | 4.58 (s) | 103.42 |
| 6"Rha-2‴ | 3.66 (br s) | 72.84 |
| 6"Rha-3‴ | 3.40 (br d, *J*=8.4 Hz) | 73.01 |
| 6"Rha-4‴ | 3.28 (dd, *J*=9.0, 8.4 Hz) | 74.68 |
| 6"Rha-5‴ | 3.38 (qd, *J*=6.0, 9.0 Hz) | 71.46 |
| 6"Rha-6‴ | 1.06 (d, *J*=6.0 Hz) | 19.35 |
| 2"Xyl-1ʺʺ | 4.83 (d, *J*=8.4 Hz) | 105.95 |
| 2"Xyl-2ʺʺ | 3.73 (dd, *J*=8.4, 9.0 Hz) | 76.96 |
| 2"Xyl-3ʺʺ | 3.58 (dd, *J*=9.0, 3.6 Hz) | 75.55 |
| 2"Xyl-4ʺʺ | 3.68 (m) | 81.12 |
| 2"Xyl-5ʺʺ | 3.44 (dd, *J*=11.4, 5.4 Hz) | 66.92 |
| | 4.20 (dd, *J*=11.4, 6.6 Hz) | |
| 4ʺʺGlc-1‴ʺ | 5.17 (d, *J*=3.6 Hz) | 102.29 |
| 4ʺʺGlc-2‴ʺ | 3.60 (dd, *J*=9.6, 3.6 Hz) | 74.68 |
| 4ʺʺGlc-3‴ʺ | 3.97 (dd, *J*=9.6, 9.6 Hz) | 76.07 |
| 4ʺʺGlc-4‴ʺ | 3.66 (m) | 79.87 |
| 4ʺʺGlc-5‴ʺ | 3.76 (m) | 73.61 |
| 4ʺʺGlc-6‴ʺ | 3.77 (m) | 63.25 |
| | 3.79 (m) | |
| 4‴ʺGlc-1‴‴ | 5.37 (d, *J*=3.6 Hz) | 102.82 |
| 4‴‴Glc-2‴‴ | 3.60 (dd, *J*=9.6, 3.6 Hz) | 74.22 |
| 4‴ʺGlc-3‴‴ | 3.68 (dd, *J*=9.0, 3.6 Hz) | 75.80 |
| 4‴ʺGlc-4‴‴ | 3.42 (m) | 72.07 |
| 4‴ʺGlc-5‴‴ | 3.46 (m) | 75.86 |
| 4‴ʺGlc-6‴‴ | 3.77 (m) | 63.14 |
| | 3.79 (m) | |

The results of nuclear magnetic resonance analysis of the kaempferol saccharide compound represented by the above Chemical Formula 6 are shown in Table 9.

**[Table 9]**

| **¹H**- **and ¹³C-NMR data of camelliaside B-G6 in D₂O** | | |
|---|---|---|
| | ¹H-NMR | ¹³C-NMR |
| 2 | | 159.98 |
| 3 | | 136.09 |
| 4 | | 180.63 |
| 5 | | 159.21 |
| 6 | 6.16 (s) | 101.86 |
| 7 | | 165.84 |
| 8 | 6.26 (br s) | 98.94 |
| 9 | | 162.89 |
| 10 | | 107.34 |
| 1' | | 124.28 |
| 2' | 7.81 (br.s) | 133.83 |
| 3' | 6.80 (br.s) | 117.92 |
| 4' | | 161.44 |
| 5' | 6.80 (br.s) | 117.92 |
| 6' | 7.81 (br.s) | 133.83 |
| 3Glc-1" | 5.29 (d, *J*=6.0 Hz) | 102.66 |
| 3Glc-2" | 3.90 (dd, *J*=6.0, 8.4 Hz) | 83.01 |
| 3Glc-3" | 3.45 (dd, *J*=9.0, 8.4 Hz) | 77.78 |
| 3Glc-4" | 3.43 (dd, *J*=9.0, 8.4 Hz) | 72.44 |
| 3Glc-5" | 3.67 (brdd, *J*=8.4, 6.0 Hz) | 78.82 |
| 3Glc-6" | 3.46 (dd, *J*=11.4, 6.0 Hz) | 69.86 |
| | 3.80 (br d, *J*=11.4 Hz) | |
| 6"Rha-1‴ | 4.57 (s) | 103.41 |
| 6"Rha-2‴ | 3.66 (br s) | 72.84 |
| 6"Rha-3‴ | 3.40 (br d, *J*=8.4 Hz) | 72.98 |
| 6"Rha-4‴ | 3.28 (dd, *J*=9.0, 8.4 Hz) | 74.68 |
| 6"Rha-5‴ | 3.38 (qd, *J*=6.0, 9.0 Hz) | 71.44 |
| 6"Rha-6‴ | 1.06 (d, *J*=6.0 Hz) | 19.31 |
| 2"Xyl-1ʺʺ | 4.83 (d, *J*=8.4 Hz) | 106.16 |
| 2"Xyl-2ʺʺ | 3.73 (dd, *J*=8.4, 9.0 Hz) | 76.89 |
| 2"Xyl-3"" | 3.58 (dd, *J*=9.0, 3.6 Hz) | 75.57 |
| 2"Xyl-4ʺʺ | 3.68 (m) | 81.00 |
| 2"Xyl-5"" | 3.44 (dd, *J*=11.4, 5.4 Hz) | 66.93 |
| | 4.20 (dd, *J*=11.4, 6.6 Hz) | |
| 4ʺʺGlc-1‴ʺ | 5.18 (d, *J*=3.6 Hz) | 102.06 |
| 4ʺʺGlc-2‴ʺ | 3.65 (dd, *J*=9.6, 3.6 Hz) | 74.63 |
| 4ʺʺGlc-3‴ʺ | 3.95 (dd, *J*=9.6, 9.6 Hz) | 75.97 |
| 4ʺʺGlc-4‴ʺ | 3.67 (m) | 79.66 |
| 4ʺʺGlc-5‴ʺ | 3.88 (m) | 74.08 |
| 4ʺʺGlc-6‴ʺ | 3.77 (m) | 63.04 |
| | 3.84 (m) | |
| 4‴ʺGlc-1‴‴ | 5.39 (d, *J*=3.6 Hz) | 102.86 |
| 4‴ʺGlc-2‴‴ | 3.65 (dd, *J*=9.6, 3.6 Hz) | 74.53 |
| 4‴ʺGlc-3‴‴ | 3.98 (dd, *J*=9.6, 9.6 Hz) | 76.25 |
| 4‴ʺGlc-4*‴‴* | 3.68 (m) | 80.32 |
| 4‴ʺGlc-5‴‴ | 3.87 (m) | 73.59 |
| 4‴ʺGlc-6‴‴ | 3.77 (m) | 63.11 |
| | 3.84 (m) | |
| 4‴‴Glc-1‴ʺʺ | 5.34 (d, *J*=3.6 Hz) | 102.80 |
| 4‴‴Glc-2‴ʺʺ | 3.60 (dd, *J*=9.6, 3.6 Hz) | 74.17 |
| 4‴‴Glc-3‴ʺʺ | 3.68 (dd, *J*=9.0, 3.6 Hz) | 75.80 |
| 4‴‴Glc-4‴ʺʺ | 3.42 (m) | 72.15 |
| 4‴‴Glc-5‴ʺʺ | 3.46 (m) | 75.86 |
| 4‴‴Glc-6‴ʺʺ | 3.77 (m) | 63.31 |
| | 3.79 (m) | |

The kaempferol heptasaccharide compound obtained by adding four sugars to the above-described Camelliaside B as a precursor may be represented by the following Chemical Formula B-1, and the results of nuclear magnetic resonance analysis of the kaempferol saccharide compound represented by Chemical Formula B-1 are shown in the below Table 10.

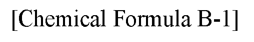

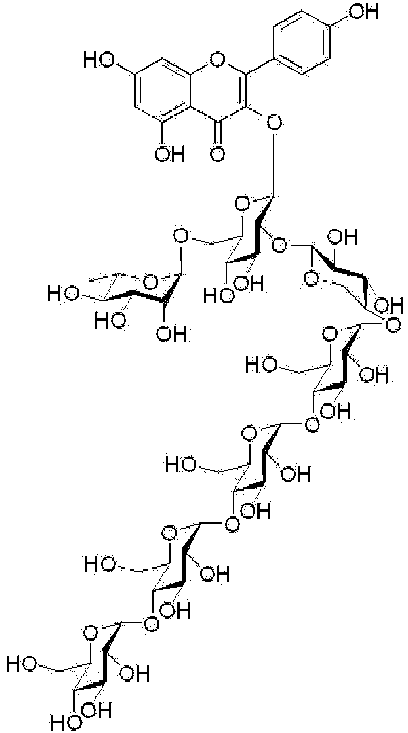

**[Table 10]**

| **¹H**- **and ¹³C-NMR data of camelliaside B-G7 in D₂O** | | |
|---|---|---|
| | ¹H-NMR | ¹³C-NMR |
| 2 | | 159.94 |
| 3 | | 136.10 |
| 4 | | 180.68 |
| 5 | | 159.30 |
| 6 | 6.20 (s) | 101.82 |
| 7 | | 165.85 |
| 8 | 6.33 (br s) | 97.42 |
| 9 | | 162.99 |
| 10 | | 107.40 |
| 1' | | 124.36 |
| 2' | 7.84 (br.s) | 133.86 |
| 3' | 6.84 (br.s) | 118.02 |
| 4' | | 161.51 |
| 5' | 6.84 (br.s) | 118.02 |
| 6' | 7.84 (br.s) | 133.86 |
| 3Glc-1" | 5.29 (d, *J*=6.0 Hz) | 102.05 |
| 3Glc-2" | 3.90 (dd, *J*=6.0, 8.4 Hz) | 83.28 |
| 3Glc-3" | 3.45 (dd, *J*=9.0, 8.4 Hz) | 77.82 |
| 3Glc-4" | 3.43 (dd, *J*=9.0, 8.4 Hz) | 72.40 |
| 3Glc-5" | 3.67 (brdd, *J*=8.4, 6.0 Hz) | 78.73 |
| 3Glc-6" | 3.51 (dd, *J*=11.4, 6.0 Hz) | 69.78 |
| | 3.82 (br d, *J*=11.4 Hz) | |
| 6"Rha-1‴ | 4.59 (s) | 103.38 |
| 6"Rha-2'" | 3.66 (br s) | 72.86 |
| 6"Rha-3‴ | 3.40 (br d, *J*=8.4 Hz) | 72.99 |
| 6"Rha-4‴ | 3.28 (dd, *J*=9.0, 8.4 Hz) | 74.75 |
| 6"Rha-5‴ | 3.38 (qd, *J*=6.0, 9.0 Hz) | 71.46 |
| 6"Rha-6‴ | 1.06 (d, *J*=6.0 Hz) | 19.31 |
| 2"Xyl-1ʺʺ | 4.79 (d, *J*=8.4 Hz) | 106.68 |
| 2"Xyl-2ʺʺ | 3.73 (dd, *J*=8.4, 9.0 Hz) | 76.95 |
| 2"Xyl-3"" | 3.58 (dd, *J*=9.0, 3.6 Hz) | 75.59 |
| 2"Xyl-4"" | 3.68 (m) | 81.00 |
| 2"Xyl-5"" | 3.38 (dd, *J*=11.4, 5.4 Hz) | 66.97 |
| | 4.18 (dd, *J*=11.4, 6.6 Hz) | |
| 4ʺʺGlc-1‴ʺ | 5.17 (d, *J*=3.6 Hz) | 102.68 |
| 4ʺʺGlc-2‴ʺ | 3.60 (dd, *J*=9.6, 3.6 Hz) | 74.54 |
| 4ʺʺGlc-3‴ʺ | 3.96 (dd, *J*=9.6, 9.6 Hz) | 76.20 |
| 4ʺʺGlc-4‴ʺ | 3.66 (m) | 80.15 |
| 4ʺʺGlc-5‴ʺ | 3.76 (m) | 74.07 |
| 4ʺʺGlc-6‴ʺ | 3.77 (m) | 63.05 |
| | 3.79 (m) | |
| 4‴ʺGlc-1‴‴ | 5.32 (d, *J*=3.6 Hz) | 102.89 |
| 4‴ʺGlc-2‴‴ | 3.65 (dd, *J*=9.6, 3.6 Hz) | 74.63 |
| 4‴ʺGlc-3‴‴ | 3.95 (dd, *J*=9.6, 9.6 Hz) | 75.95 |
| 4‴ʺGlc-4*‴‴* | 3.67 (m) | 80.20 |
| 4‴ʺGlc-5‴‴ | 3.88 (m) | 73.61 |
| 4‴ʺGlc-6‴‴ | 3.77 (m) | 62.98 |
| | 3.84 (m) | |
| 4‴‴Glc-1‴ʺʺ | 5.38 (d, *J*=3.6 Hz) | 103.00 |
| 4‴‴Glc-2‴ʺʺ | 3.65 (dd, *J*=9.6, 3.6 Hz) | 74.64 |
| 4‴‴Glc-3‴ʺʺ | 3.98 (dd, *J*=9.6, 9.6 Hz) | 76.21 |
| 4‴‴Glc-4‴ʺʺ | 3.68 (m) | 80.60 |
| 4‴‴Glc-5‴ʺʺ | 3.87 (m) | 74.12 |
| 4‴‴Glc-6‴ʺʺ | 3.77 (m) | 63.25 |
| | 3.85 (m) | |
| 4‴ʺʺGlc-1‴‴ʺ | 5.36(d, *J*=3.6 Hz) | 103.06 |
| 4‴ʺʺGlc-2‴‴ʺ | 3.60 (dd, *J*=9.6, 3.6 Hz) | 74.14 |
| 4‴ʺʺGlc-3‴‴ʺ | 3.68 (dd, *J*=9.0, 3.6 Hz) | 75.83 |
| 4‴ʺʺGlc-4‴‴ʺ | 3.42 (m) | 72.11 |
| 4‴ʺʺGlc-5‴‴ʺ | 3.46 (m) | 75.87 |
| 4‴ʺʺGlc-6‴‴ʺ | 3.78 (m) | 63.30 |
| | 3.86 (m) | |

In one embodiment of the present disclosure, Nicotiflorin may be used as a precursor, and Nicotiflorin may be represented by the following Chemical Formula N. The results of nuclear magnetic resonance analysis of the kaempferol saccharide compound represented by Chemical Formula N are shown in Table 11.

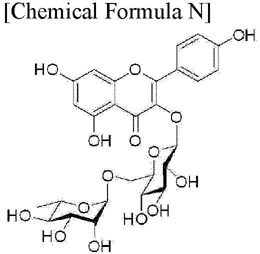

**[Table 11]**

| **¹H- and ¹³C-NMR data of nicotiflorin in DMSO-*d*₆** | | |
|---|---|---|
| | ¹H-NMR | ¹³C-NMR |
| 2 | | 158.43 |
| 3 | | 134.95 |
| 4 | | 178.85 |
| 5 | | 158.31 |
| 6 | 6.16 (s) | 101.95 |
| 7 | | 166.45 |
| 8 | 6.36 (br s) | 95.76 |
| 9 | | 162.89 |
| 10 | | 105.09 |
| 1' | | 124.34 |
| 2' | 7.99 (d, *J*=9.0 Hz) | 132.61 |
| 3' | 6.89 (d, *J*=9.0 Hz) | 116.84 |
| 4' | | 161.65 |
| 5' | 6.89 (d, *J*=9.0 Hz) | 116.84 |
| 6' | 7.99 (d, *J*=9.0 Hz) | 132.61 |
| 3Glc-1" | 5.30 (d, *J*=7.8 Hz) | 103.33 |
| 3Glc-2" | 3.18 (dd, *J*=7.8, 9.0 Hz) | 75.97 |
| 3Glc-3" | 3.23 (dd, *J*=9.0, 9.0 Hz) | 78.12 |
| 3Glc-4" | 3.06 (dd, *J*=9.0, 9.0 Hz) | 71.63 |
| 3Glc-5" | 3.26 (brdd, *J*=9.0, 6.0 Hz) | 77.48 |
| 3Glc-6" | 3.28 (dd, *J*=9.6, 6.0 Hz) | 68.69 |
| | 3.71 (br d, *J*=9.6 Hz) | |
| 6"Rha-1‴ | 4.39 (d, *J*=1.2 Hz) | 102.56 |
| 6"Rha-2‴ | 3.44 (dd, *J*=1.2, 3.0 Hz) | 72.32 |
| 6"Rha-3‴ | 3.30 (dd, *J*=9.0, 3.0 Hz) | 72.08 |
| 6"Rha-4‴ | 3.11 (dd, *J*=9.0, 9.0 Hz) | 73.55 |
| 6"Rha-5‴ | 3.29 (qd, *J*=6.0, 9.0 Hz) | 70.04 |
| 6"Rha-6‴ | 1.01 (d, *J*=6.0 Hz) | 19.54 |

The results of nuclear magnetic resonance analysis of the kaempferol saccharide compound represented by the following Chemical Formula N-1 prepared using the compound represented by the above-described Chemical Formula N as a precursor are shown in Table 12.

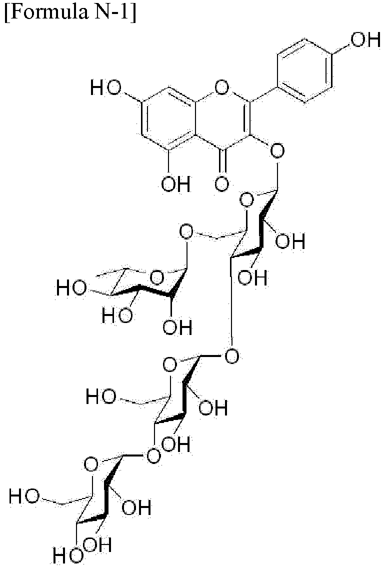

**[Table 12]**

| **¹H**- **and ¹³C-NMR data of nicotiflorin-G4 in D₂O** | | |
|---|---|---|
| | ¹H-NMR | ¹³C-NMR |
| 2 | | 160.17 |
| 3 | | 136.82 |
| 4 | | 180.02 |
| 5 | | 158.80 |
| 6 | 6.01 (s) | 101.58 |
| 7 | | 165.97 |
| 8 | 5.88 (br s) | 97.25 |
| 9 | | 162.64 |
| 10 | | 106.81 |
| 1' | | 123.71 |
| 2' | 7.59 (br.s) | 133.76 |
| 3' | 6.72 (brs) | 117.89 |
| 4' | | 161.61 |
| 5' | 6.72 (br.s) | 117.89 |
| 6' | 7.59 (br.s) | 133.87 |
| 3Glc-1" | 4.67 (d, *J*=6.6 Hz) | 106.08 |
| 3Glc-2" | 3.52 (dd, *J*=6.6, 8.4 Hz) | 76.30 |
| 3Glc-3" | 3.63 (dd, *J*=9.0, 8.4 Hz) | 78.85 |
| 3Glc-4" | 3.63 (dd, *J*=9.0, 8.4 Hz) | 80.54 |
| 3Glc-5" | 3.32 (brdd, *J*=8.4, 6.0 Hz) | 76.23 |
| 3Glc-6" | 3.44 (dd, *J*=11.4, 6.0 Hz) | 69.46 |
| | 3.62 (br d, *J*=11.4 Hz) | |
| 6"Rha-1‴ | 4.61 (s) | 103.47 |
| 6"Rha-2‴ | 3.77 (br s) | 73.12 |
| 6"Rha-3‴ | 3.77 (br d, *J*=8.4 Hz) | 72.95 |
| 6"Rha-4‴ | 3.37 (dd, *J*=9.0, 8.4 Hz) | 74.77 |
| 6"Rha-5‴ | 3.38 (qd, *J*=6.0, 9.0 Hz) | 71.55 |
| 6"Rha-6‴ | 1.11 (d, *J*=6.0 Hz) | 19.67 |
| 4"Glc-1ʺʺ | 5.28 (d, *J*=3.6 Hz) | 104.75 |
| 4"Glc-2ʺʺ | 3.62 (dd, *J*=9.0, 3.6 Hz) | 74.73 |
| 4"Glc-3ʺʺ | 3.95 (dd, *J*=9.0, 9.0 Hz) | 76.23 |
| 4"Glc-4ʺʺ | 3.67 (dd, *J*=9.0, 9.0 Hz) | 79.95 |
| 4"Glc-5ʺʺ | 3.41 (m) | 74.13 |
| 4"Glc-6ʺʺ | 3.73 (m) | 63.07 |
| | 3.84 (m) | |
| 4ʺʺGlc-1‴ʺ | 5.37 (d, *J*=3.6 Hz) | 102.49 |
| 4ʺʺGlc-2‴ʺ | 3.58 (dd, *J*=9.0, 3.6 Hz) | 74.56 |
| 4ʺʺGlc-3‴ʺ | 3.69 (dd, *J*=9.0, 9.0 Hz) | 75.63 |
| 4ʺʺGlc-4‴ʺ | 3.42 (dd, *J*=9.0, 9.0 Hz) | 72.16 |
| 4ʺʺGlc-5‴ʺ | 3.70 (m) | 75.81 |
| 4ʺʺGlc-6‴ʺ | 3.74 (m) | 63.33 |
| | 3.85 (m) | |

The results of nuclear magnetic resonance analysis of the kaempferol saccharide compound represented by the following Chemical Formula N-2 prepared using the compound represented by the above-described Chemical Formula N as a precursor are shown in Table 13.

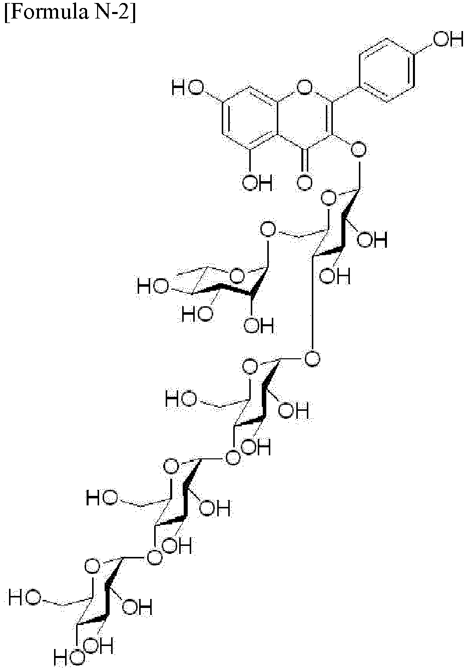

**[Table 13]**

| **¹H**- **and ¹³C-NMR data of nicotiflorin-G5 in D₂O** | | |
|---|---|---|
| | ¹H-NMR | ¹³C-NMR |
| 2 | | 160.40 |
| 3 | | 136.88 |
| 4 | | 180.01 |
| 5 | | 158.96 |
| 6 | 6.05 (s) | 101.58 |
| 7 | | 166.03 |
| 8 | 6.00 (br s) | 101.78 |
| 9 | | 162.70 |
| 10 | | 106.92 |
| 1' | | 123.84 |
| 2' | 7.66 (br.s) | 133.85 |
| 3' | 6.77 (br.s) | 118.01 |
| 4' | | 161.62 |
| 5' | 6.72 (br.s) | 118.01 |
| 6' | 7.66 (br.s) | 133.85 |
| 3Glc-1" | 4.71 (d, *J*=6.6 Hz) | 106.05 |
| 3Glc-2" | 3.54 (dd, *J*=6.6, 8.4 Hz) | 76.34 |
| 3Glc-3" | 3.63 (dd, *J*=9.0, 8.4 Hz) | 78.90 |
| 3Glc-4" | 3.63 (dd, *J*=9.0, 8.4 Hz) | 80.40 |
| 3Glc-5" | 3.32 (brdd, *J*=8.4, 6.0 Hz) | 76.21 |
| 3Glc-6" | 3.44 (dd, *J*=11.4, 6.0 Hz) | 69.58 |
| | 3.62 (br d, *J*=11.4 Hz) | |
| 6"Rha-1‴ | 4.61 (s) | 103.45 |
| 6"Rha-2‴ | 3.78 (br s) | 73.14 |
| 6"Rha-3‴ | 3.77 (br d, *J*=8.4 Hz) | 72.94 |
| 6"Rha-4‴ | 3.37 (dd, *J*=9.0, 8.4 Hz) | 74.75 |
| 6"Rha-5‴ | 3.38 (qd, *J*=6.0, 9.0 Hz) | 71.55 |
| 6"Rha-6‴ | 1.11 (d, *J*=6.0 Hz) | 19.73 |
| 4"Glc-1ʺʺ | 5.28 (d, *J*=3.6 Hz) | 103.07 |
| 4"Glc-2ʺʺ | 3.63 (dd, *J*=9.0, 3.6 Hz) | 74.73 |
| 4"Glc-3ʺʺ | 3.95 (dd, *J*=9.0, 9.0 Hz) | 76.17 |
| 4"Glc-4ʺʺ | 3.66 (dd, *J*=9.0, 9.0 Hz) | 80.17 |
| 4"Glc-5ʺʺ | 3.41 (m) | 74.16 |
| 4"Glc-6ʺʺ | 3.73 (m) | 63.04 |
| | 3.84 (m) | |
| 4ʺʺGlc-1‴ʺ | 5.28 (d, *J*=3.6 Hz) | 103.03 |
| 4ʺʺGlc-2‴ʺ | 3.63 (dd, *J*=9.0, 3.6 Hz) | 74.46 |
| 4ʺʺGlc-3‴ʺ | 3.95 (dd, *J*=9.0, 9.0 Hz) | 76.17 |
| 4ʺʺGlc-4‴ʺ | 3.67 (dd, *J*=9.0, 9.0 Hz) | 80.34 |
| 4ʺʺGlc-5‴ʺ | 3.40 (m) | 74.16 |
| 4ʺʺGlc-6‴ʺ | 3.73 (m) | 63.26 |
| | 3.84 (m) | |
| 4‴ʺGlc-1‴‴ | 5.37 (d, *J*=3.6 Hz) | 102.94 |
| 4‴ʺGlc-2‴‴ | 3.54 (dd, *J*=9.0, 3.6 Hz) | 74.56 |
| 4‴ʺGlc-3‴‴ | 3.68 (dd, *J*=9.0, 9.0 Hz) | 75.59 |
| 4‴ʺGlc-4‴‴ | 3.42 (dd, *J*=9.0, 9.0 Hz) | 72.13 |
| 4‴ʺGlc-5‴‴ | 3.71 (m) | 75.82 |
| 4‴ʺGlc-6‴‴ | 3.73 (m) | 63.32 |
| | 3.86 (m) | |

The results of nuclear magnetic resonance analysis of the kaempferol saccharide compound represented by the following Chemical Formula N-3 prepared using the compound represented by the above-described Chemical Formula N as a precursor are shown in Table 14.

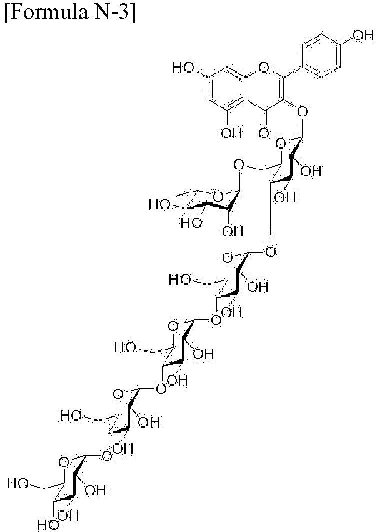

**[Table 14]**

| **¹H- and ¹³C-NMR data of nicotiflorin-G6 in D₂O** | | |
|---|---|---|
| | ¹H-NMR | ¹³C-NMR |
| 2 | | 160.52 |
| 3 | | 136.88 |
| 4 | | 180.25 |
| 5 | | 159.04 |
| 6 | 6.09 (s) | 101.82 |
| 7 | | 166.11 |
| 8 | 6.09 (br s) | 97.42 |
| 9 | | 162.74 |
| 10 | | 106.97 |
| 1' | | 123.93 |
| 2' | 7.73 (br.s) | 133.89 |
| 3' | 6.80 (br.s) | 118.00 |
| 4' | | 161.62 |
| 5' | 6.80 (br.s) | 118.00 |
| 6' | 7.73 (br.s) | 133.89 |
| 3Glc-1" | 4.76 (d, *J*=6.6 Hz) | 105.96 |
| 3Glc-2" | 3.54 (dd, *J*=6.6, 8.4 Hz) | 76.34 |
| 3Glc-3" | 3.63 (dd, *J*=9.0, 8.4 Hz) | 78.91 |
| 3Glc-4" | 3.63 (dd, *J*=9.0, 8.4 Hz) | 80.57 |
| 3Glc-5" | 3.32 (brdd, *J*=8.4, 6.0 Hz) | 76.20 |
| 3Glc-6" | 3.44 (dd, *J*=11.4, 6.0 Hz) | 69.59 |
| | 3.62 (br d, *J*=11.4 Hz) | |
| 6"Rha-1‴ | 4.59 (s) | 103.38 |
| 6"Rha-2‴ | 3.78 (br s) | 73.14 |
| 6"Rha-3‴ | 3.77 (br d, *J*=8.4 Hz) | 72.94 |
| 6"Rha-4‴ | 3.37 (dd, *J*=9.0, 8.4 Hz) | 74.78 |
| 6"Rha-5‴ | 3.38 (qd, *J*=6.0, 9.0 Hz) | 71.55 |
| 6''Rha-6‴ | 1.14 (d, *J*=6.0 Hz) | 19.73 |
| 4"Glc-1ʺʺ | 5.29 (d, *J*=3.6 Hz) | 103.08 |
| 4"Glc-2ʺʺ | 3.63 (dd, *J*=9.0, 3.6 Hz) | 74.47 |
| 4"Glc-3ʺʺ | 3.95 (dd, *J*=9.0, 9.0 Hz) | 76.10 |
| 4"Glc-4ʺʺ | 3.66 (dd, *J*=9.0, 9.0 Hz) | 80.11 |
| 4"Glc-5ʺʺ | 3.41 (m) | 74.37 |
| | 3.73 (m) | 63.01 |
| 4"Glc-6ʺʺ | 3.84 (m) | |
| 4ʺʺGlc-1‴ʺ | 5.30 (d, *J*=3.6 Hz) | 102.92 |
| 4ʺʺGlc-2‴ʺ | 3.63 (dd, *J*=9.0, 3.6 Hz) | 74.65 |
| 4ʺʺGlc-3‴ʺ | 3.95 (dd, *J*=9.0, 9.0 Hz) | 76.10 |
| 4ʺʺGlc-4‴ʺ | 3.67 (dd, *J*=9.0, 9.0 Hz) | 80.20 |
| 4ʺʺGlc-5‴ʺ | 3.40 (m) | 74.12 |
| 4ʺʺGlc-6‴ʺ | 3.73 (m) | 63.16 |
| | 3.84 (m) | |
| 4‴ʺGlc-1‴‴ | 5.30 (d, *J*=3.6 Hz) | 102.92 |
| 4‴ʺGlc-2‴‴ | 3.63 (dd, *J*=9.0, 3.6 Hz) | 74.44 |
| 4‴ʺGlc-3‴‴ | 3.95 (dd, *J*=9.0, 9.0 Hz) | 76.13 |
| 4‴ʺGlc-4‴‴ | 3.67 (dd, *J*=9.0, 9.0 Hz) | 80.43 |
| 4‴ʺGlc-5‴‴ | 3.40 (m) | 74.12 |
| 4‴ʺGlc-6‴‴ | 3.73 (m) | 63.24 |
| | 3.84 (m) | |
| 4‴‴Glc-1‴ʺʺ | 5.33 (d, *J*=3.6 Hz) | 102.92 |
| 4‴‴Glc-2‴ʺʺ | 3.54 (dd, *J*=9.0, 3.6 Hz) | 74.44 |
| 4‴‴Glc-3‴ʺʺ | 3.68 (dd, *J*=9.0, 9.0 Hz) | 75.58 |
| 4‴‴Glc-4‴ʺʺ | 3.42 (dd, *J*=9.0, 9.0 Hz) | 72.15 |
| 4‴‴Glc-5‴ʺʺ | 3.71 (m) | 75.75 |
| 4‴‴Glc-6‴ʺʺ | 3.73 (m) | 63.33 |
| | 3.86 (m) | |

The results of nuclear magnetic resonance analysis of the kaempferol saccharide compound represented by the following Chemical Formula N-4 prepared using the compound represented by the above-described Chemical Formula N as a precursor are shown in Table 15.

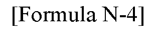

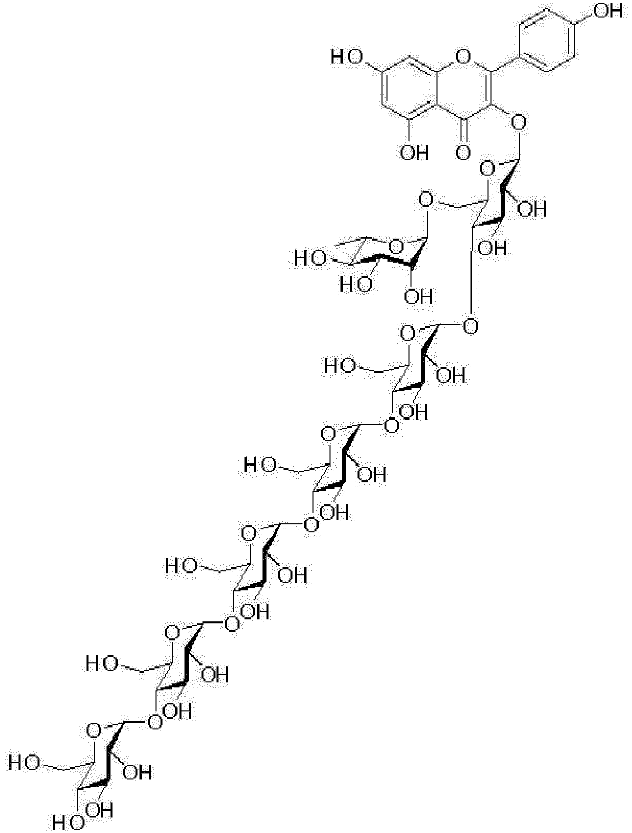

**[Table 15]**

| **¹H- and ¹³C-NMR data of nicotiflorin-G7 in D₂O** | | |
|---|---|---|
| | ¹H-NMR | ¹³C-NMR |
| 2 | | 161.61 |
| 3 | | 136.82 |
| 4 | | 179.45 |
| 5 | | 159.69 |
| 6 | 6.09 (s) | 101.55 |
| 7 | | 166.48 |
| 8 | 6.09 (br s) | 97.42 |
| 9 | | 164.39 |
| 10 | | 107.14 |
| 1' | | 123.29 |
| 2' | 7.73 (br.s) | 133.98 |
| 3' | 6.80 (br.s) | 118.04 |
| 4' | | 161.79 |
| 5' | 6.80 (br.s) | 118.04 |
| 6' | 7.73 (br.s) | 133.98 |
| 3Glc-1" | 4.76 (d, *J*=6.6 Hz) | 105.96 |
| 3Glc-2" | 3.54 (dd, *J*=6.6, 8.4 Hz) | 76.35 |
| 3Glc-3" | 3.63 (dd, *J*=9.0, 8.4 Hz) | 78.91 |
| 3Glc-4" | 3.63 (dd, *J*=9.0, 8.4 Hz) | 80.86 |
| 3Glc-5" | 3.32 (brdd, *J*=8.4, 6.0 Hz) | 76.17 |
| 3Glc-6" | 3.44 (dd, *J*=11.4, 6.0 Hz) | 69.79 |
| | 3.62 (br d, *J*=11.4 Hz) | |
| 6"Rha-1'" | 4.59 (s) | 103.00 |
| 6"Rha-2‴ | 3.78 (br s) | 73.09 |
| 6"Rha-3‴ | 3.77 (br d, *J*=8.4 Hz) | 72.91 |
| 6"Rha-4‴ | 3.37 (dd, *J*=9.0, 8.4 Hz) | 74.70 |
| 6"Rha-5‴ | 3.38 (qd, *J*=6.0, 9.0 Hz) | 71.53 |
| 6"Rha-6'" | 1.14 (d, *J*=6.0 Hz) | 19.73 |
| 4"Glc-1"" | 5.29 (d, *J*=3.6 Hz) | 102.09 |
| 4"Glc-2ʺʺ | 3.63 (dd, *J*=9.0, 3.6 Hz) | 74.62 |
| 4"Glc-3ʺʺ^{.} | 3.95 (dd, *J*=9.0, 9.0 Hz) | 75.74 |
| 4"Glc-4"" | 3.66 (dd, *J*=9.0, 9.0 Hz) | 79.55 |
| 4"Glc-5ʺʺ | 3.41 (m) | 74.07 |
| 4"Glc-6"" | 3.73 (m) | 63.05 |
| | 3.84 (m) | |
| 4ʺʺGlc-1‴ʺ | 5.30 (d, *J*=3.6 Hz) | 102.60 |
| 4ʺʺGlc-2‴ʺ | 3.63 (dd, *J*=9.0, 3.6 Hz) | 74.43 |
| 4ʺʺGlc-3‴ʺ | 3.95 (dd, *J*=9.0, 9.0 Hz) | 76.16 |
| 4ʺʺGlc-4‴ʺ | 3.67 (dd, *J*=9.0, 9.0 Hz) | 80.29 |
| 4ʺʺGlc-5‴ʺ | 3.40 (m) | 74.07 |
| 4ʺʺGlc-6‴ʺ | 3.73 (m) | 63.08 |
| | 3.84 (m) | |
| 4‴ʺGlc-1‴‴ | 5.30 (d, *J*=3.6 Hz) | 102.71 |
| 4‴ʺGlc-2‴‴ | 3.63 (dd, *J*=9.0, 3.6 Hz) | 74.43 |
| 4‴ʺGlc-3‴‴ | 3.95 (dd, *J*=9.0, 9.0 Hz) | 75.77 |
| 4‴ʺGlc-4‴‴ | 3.67 (dd, *J*=9.0, 9.0 Hz) | 80.14 |
| 4‴ʺGlc-5‴‴ | 3.40 (m) | 74.06 |
| 4‴ʺGlc-6‴‴ | 3.73 (m) | 63.19 |
| | 3.84 (m) | |
| 4‴‴Glc-1‴ʺʺ | 5.30 (d, *J*=3.6 Hz) | 102.71 |
| 4‴‴Glc-2‴ʺʺ | 3.63 (dd, *J*=9.0, 3.6 Hz) | 74.11 |
| 4‴‴Glc-3‴ʺʺ | 3.95 (dd, *J*=9.0, 9.0 Hz) | 76.12 |
| 4‴‴Glc-4‴ʺʺ | 3.67 (dd, *J*=9.0, 9.0 Hz) | 80.24 |
| 4‴‴Glc-5‴ʺʺ | 3.40 (m) | 74.06 |
| 4‴‴Glc-6‴ʺʺ | 3.73 (m) | 63.32 |
| | 3.84 (m) | |
| 4‴ʺʺGlc-1‴‴ʺ | 5.33 (d, *J*=3.6 Hz) | 102.79 |
| 4‴ʺʺGlc-2‴‴ʺ | 3.54 (dd, *J*=9.0, 3.6 Hz) | 74.11 |
| 4‴ʺʺGlc-3‴‴ʺ | 3.68 (dd, *J*=9.0, 9.0 Hz) | 75.58 |
| 4‴ʺʺGlc-4‴‴ʺ | 3.42 (dd, *J*=9.0, 9.0 Hz) | 72.19 |
| 4‴ʺʺGlc-5‴‴ʺ | 3.71 (m) | 75.61 |
| 4‴ʺʺGlc-6‴‴ʺ | 3.73 (m) | 63.34 |
| | 3.86 (m) | |

The composition according to one aspect of the present disclosure may realize reaction conditions in which kaempferol 4-6 saccharide compounds are produced by adding glucose using kaempferol trisaccharides extracted from *Camellia oleifera* seed cake, which is discarded after oil extraction from camellia seeds, that is, a compound represented by Chemical Formula A or Chemical Formula B as a precursor.

Compared to a composition of kaempferol trisaccharide compound extracted from *Camellia oleifera* seed cake, it may exhibit significantly superior skin protection.

*Camellia oleifera* seed cake extract consists of terpine saccharides, flavonoid saccharides, sugars, and proteins. In particular, the flavonoid saccharides of *Camellia oleifera* seed cake are composed of trisaccharide compounds (Chemical Formula A, Chemical Formula B). In the present disclosure, it was confirmed that eight types of 4-6 saccharides (Chemical Formula 1-6), in which glucose is sequentially linked using kaempferol trisaccharide compounds derived from *Camellia oleifera* seed cake as precursors, have an excellent skin protection effect.

In one embodiment, the enzyme-treated-kaempferol saccharide compound may be obtained by enzymatically treating kaempferol trisaccharide compound.

In one embodiment, the kaempferol trisaccharide compound may be an extract derived from *Camellia oleifera.*

Specifically, the extract and kaempferol trisaccharide compound may be obtained by extracting the kaempferol saccharide compound using 70% ethanol from *Camellia oleifera* seed cake after producing oil from *Camellia oleifera* seeds through a pressing method.

In one embodiment, the enzyme may be cyclodextrin glucanotransferase.

Exemplary embodiments of the present disclosure provide a composition for protecting skin, comprising an enzyme-treated-kaempferol saccharide compound, a stereoisomer thereof, a pharmaceutically acceptable salt thereof, a hydrate thereof, or a solvate thereof as an active ingredient.

In one embodiment, the daily application amount of the active ingredient may be 1 to 1000 mg/Kg. For example, the daily application amount of the active ingredient may be 1 mg/Kg or more, 5 mg/Kg or more, 10 mg/Kg or more, 50 mg/Kg or more, 100 mg/Kg or more, 300 mg/Kg or more, 500 mg/Kg or more. , 700 mg/Kg or more, or 900 mg/Kg or more, and may be 1000 mg/Kg or less, 800 mg/Kg or less, 600 mg/Kg or less, 400 mg/Kg or less, 200 mg/Kg or less, 100 mg/Kg, 50 mg/Kg or less, 10 mg/Kg or less, or 5 mg/Kg or less.

If the daily application amount of the active ingredient is less than 1 mg/Kg, the skin protection effect may be minimal, and if it is more than 1000 mg/Kg, cytotoxicity may occur.

In one embodiment, the composition can inhibit the activity of 3-Phosphoinositide-dependent kinase 1 (PDK1).

According to Korean Patent Publication No. 10-2022-0036148, when PDK1 activity is inhibited, cells whose growth has been suppressed due to aging can grow again, and damaged cells can also recover. Specifically, inhibition of PDK1 in aged human dermal fibroblasts can eradicate aging characteristics and inhibit NF-κB and mTOR signaling, restoring the cell production cycle and restoring or promoting skin regeneration ability. Additionally, PDK1 inhibition reverses dermal aging associated with replicative senescence and chemically induced senescence, as evidenced by increased MMP1, decreased collagen fibers, and decreased procollagen secretion. For epidermal homeostasis, the balance between keratinocyte proliferation and differentiation is regulated by dermal fibroblasts. Reversal of aging dermal fibroblasts by PDK1 inhibition affects keratinocyte proliferation and differentiation. PDK1 inhibition restores the reduced abundance of these keratinocyte differentiation markers and components of the dermoepidermal junction found in skin equivalents of aged NHDFs.

According to Korean Patent Publication No. 10-2021-0037643, PDK1 is a protein that simultaneously regulates mTOR and NF-xB among the major signaling proteins involved in cellular aging, and is a key protein that determines cell growth, division, and aging-related secretory phenotype. When PDK1 is temporarily inhibited, some of the aged cells are effectively reverted to normal young cells, and the aged cell phenotype is no longer observed in these cells. PDK1 inhibitors inhibit the transcriptional activity of mTOR (mammalian Target Of Rapamycin) and NF-κB (Nuclear Factor Kappa B). Inhibiting PDK may induce reverse aging.

In one embodiment, the composition may be for improving skin wrinkles.

In one embodiment, the composition may be for promoting skin regeneration.

In one embodiment, the composition may be for improving atopic skin.

In one embodiment, the composition may be for regulating the skin keratin production cycle.

According to one aspect of the present disclosure, the composition may shorten the skin keratin production cycle or the turnover cycle of epidermal cells by promoting colony formation and differentiation of epidermal cells.

In another embodiment, the composition may be for regulating skin keratin, promoting skin keratin turnover, or normalizing the skin keratin turnover cycle.

In another embodiment, the composition may be for improving skin wrinkles by inhibiting the activity of 3-Phosphoinositide-dependent kinase 1 (PDK1).

In another embodiment, the composition may be for promoting skin regeneration by inhibiting the activity of 3-Phosphoinositide-dependent kinase 1 (PDK1).

In another embodiment, the composition may be for improving atopic skin, or improving, preventing or treating atopic dermatitis by inhibiting the activity of 3-Phosphoinositide-dependent kinase 1 (PDK1).

In another embodiment, the composition may be for regulating the skin keratin production cycle by inhibiting the activity of 3-Phosphoinositide-dependent kinase 1 (PDK1).

In another embodiment, the composition may be for regulating skin keratin by inhibiting the activity of 3-Phosphoinositide-dependent kinase 1 (PDK1).

In another embodiment, the composition may be for promoting skin keratin turnover by inhibiting the activity of 3-Phosphoinositide-dependent kinase 1 (PDK1).

In another embodiment, the composition may be for normalizing the skin keratin turnover cycle by inhibiting the activity of 3-Phosphoinositide-dependent kinase 1 (PDK1).

In one embodiment, the composition may be for improving skin sagging.

In one embodiment, the composition may be for increasing the expression of Col7Al.

In one embodiment, the composition may be for increasing the expression of Coll7Al.

The Col7Al and Coll7Al are proteins that constitute DEJ (dermoepidermal junction). A decrease in Col7Al and Coll7Al impairs the function of DEJ, which holds epidermal cells firmly and strengthens the bond between the epidermal layer and the dermal layer, thereby causing skin sagging. Therefore, restoration of reduced Col7Al and Coll7Al expression can prevent and improve skin sagging by preventing and improving DEJ and rete ridges structural deformation.

According to Korean Patent Publication No. 10-2022-0036148, as a result of investigating whether reversal of aging dermal fibroblasts by PDK1 inhibition affects the proliferation and differentiation of keratinocytes, it was confirmed that inhibition of PDK1 restored decreased dermal-epidermal junction components such as COL17A1.

In another embodiment, the composition may be for improving skin sagging by inhibiting the activity of 3-Phosphoinositide-dependent kinase 1 (PDK1).

In another embodiment, the composition may be for increasing the expression of Col7Al by inhibiting the activity of 3-Phosphoinositide-dependent kinase 1 (PDK1).

In another embodiment, the composition may be for increasing the expression of Coll7Al by inhibiting the activity of 3-Phosphoinositide-dependent kinase 1 (PDK1).

In one embodiment, the composition may be for improving skin barrier function.

In one embodiment, the composition may be for improving skin moisturization.

In one embodiment, the composition may be for increasing the expression of keratin 10.

In one embodiment, the composition may be for increasing the expression of filaggrin.

Keratin 10 and filaggrin are genes that normalize epidermal keratinization by regulating keratinization of skin cells, and are known to be involved in skin barrier function, skin moisturization, skin wrinkles, and aging, etc. Therefore, increased expression of keratin 10 and filaggrin can promote moisture retention in skin tissue and strengthen the skin barrier that prevents loss of moisture in skin tissue.

According to Korean Patent Publication No. 10-2022-0036148, as a result of investigating whether reversal of aging dermal fibroblasts by PDK1 inhibition affects the proliferation and differentiation of keratinocytes, it was confirmed that inhibition of PDK1 restored decreased expressions of keratinocyte differentiation markers such as keratin 10 and filaggrin.

In another embodiment, the composition may be for improving skin barrier function by inhibiting the activity of 3-Phosphoinositide-dependent kinase 1 (PDK1).

In another embodiment, the composition may be for improving skin moisturization by inhibiting the activity of 3-Phosphoinositide-dependent kinase 1 (PDK1).

In another embodiment, the composition may be for increasing the expression of keratin 10 by inhibiting the activity of 3-Phosphoinositide-dependent kinase 1 (PDK1).

In another embodiment, the composition may be for increasing the expression filaggrin by inhibiting the activity of 3-Phosphoinositide-dependent kinase 1 (PDK1).

In one embodiment, the composition may be a cosmetic composition.

The appearance of the cosmetic composition according to an embodiment of the present disclosure may contain a cosmetically or dermatologically acceptable medium or base. It may be provided in any formulation suitable for topical application, for example in the form of solutions, gels, solids, pasty anhydrous products, emulsions obtained by dispersing the oil phase in the water phase, suspensions, microemulsions, microcapsules, microgranules or ionic forms (liposomes) and non-ionic vesicular dispersants, or in the form of creams, skins, lotions, powders, ointments, sprays or conceal sticks. These compositions may be prepared according to conventional methods in the art. The composition according to the present disclosure may also be used in the form of a foam or in the form of an aerosol composition further containing a compressed propellant.

The cosmetic composition according to an embodiment of the present disclosure is not particularly limited in its formulation, and may be formulated into cosmetics such as softening lotion, astringent lotion, nourishing lotion, nourishing cream, massage cream, essence, eye cream, eye essence, cleansing cream, cleansing foam, cleansing water, pack, powder, body lotion, body cream, body oil, and body essence, etc.

When the formulation according to an embodiment of the present disclosure is a paste, cream, or gel, animal fiber, plant fiber, wax, paraffin, starch, tracant, cellulose derivative, polyethylene glycol, silicone, bentonite, silica, talc, or oxidation zine, etc. may be used as the carrier ingredient.

When the formulation according to an embodiment of the present disclosure is a powder or spray, lactose, talc, silica, aluminum hydroxide, calcium silicate, or polyamide powder may be used as the carrier ingredient, and especially in the case of a spray, propellants such as chlorofluorohydrocarbon, propane/butane or dimethyl ether may be used as carrier ingredients.

When the formulation according to an embodiment of the present disclosure is a solution or emulsion, a solvent, solvating agent, or emulsifying agent such as water, ethanol, isopropanol, ethyl carbonate, ethyl acetate, benzyl alcohol, benzyl benzoate, propylene glycol, 1,3-butyl glycol oil, glycerol fatty esters, polyethylene glycol or fatty acid esters of sorbitan is used as a carrier ingredient.

When the formulation according to an embodiment of the present disclosure is a suspension, a liquid diluent such as water, ethanol or propylene glycol, a suspension such as ethoxylated isostearyl alcohol, polyoxyethylene sorbitol ester and polyoxyethylene sorbitan ester, and. microcrystalline cellulose, aluminum metahydroxide, bentonite, agar, or tracant may be used as a carrier ingredient.

When the formulation according to an embodiment of the present disclosure is a surfactant-containing cleansing agent, aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, fatty alcohol, fatty acid glyceride, fatty acid diethanolamide, vegetable oil, linoline derivative, or ethoxylated glycerol fatty acid ester may be used as a carrier ingredient.

The cosmetic composition according to an embodiment of the present disclosure may further include functional additives and components included in general cosmetic compositions in addition to the active ingredients. The functional additive may include ingredients selected from the group consisting of water-soluble vitamins, oil-soluble vitamins, polymer peptides, polymer polysaccharides, sphingolipids, and seaweed extract.

In addition to the functional additives described above, the cosmetic composition according to an embodiment of the present disclosure may also contain ingredients included in general cosmetic compositions, if necessary. Other ingredients included include oils and fats, moisturizers, emollients, surfactants, organic and inorganic pigments, organic powders, ultraviolet absorbers, preservatives, disinfectants, antioxidants, plant extracts, pH adjusters, alcohol, pigments, fragrances, blood circulation accelerators, cooling agents, restrictors, and purified water, etc.

In addition, the composition according to an embodiment of the present disclosure may be a skin external preparation, and the skin external preparation is a general term that can include anything applied outside the skin, and various formulations of cosmetics and pharmaceuticals may be included here.

In one embodiment, the composition may be a non-therapeutic transdermal composition.

In one embodiment, the composition may be a food composition for improving skin moisture status.

In one embodiment, the composition may be an oral composition.

In one embodiment, the composition may be a non-therapeutic oral composition.

The food composition or oral composition according to an embodiment of the present disclosure may be in a liquid or solid formulation, and may be in the form of tablets, capsules, soft capsules, pills, granules, beverages (drinks), diet bars, chocolate, and caramel formulations, and the formulation is not particularly limited.

In addition to the above active ingredients, the food composition or oral composition of the present disclosure may contain excipients, sugars, flavorings, pigments, oils and fats, proteins, etc. as necessary.

Exemplary embodiments of the present disclosure provide a method for manufacturing the enzyme-treated-kaempferol saccharide compound, comprising: obtaining a kaempferol saccharide compound from *Camellia oleifera* seed cake; purifying the kaempferol saccharide compound; enzymatically treating the purified kaempferol saccharide compound; and purifying the enzyme-treated enzyme-treated-kaempferol saccharide compound.

In one embodiment, the purified kaempferol saccharide compound may be a kaempferol trisaccharide compound.

In one embodiment, the enzyme may be cyclodextrin glucanotransferase.

Hereinafter, the present invention will be described in more detail through the following examples. However, the following examples are provided only for illustrative purposes to aid understanding of the present invention, and the scope and scope of the present invention are not limited thereto.

### Example

The *Camellia oleifera* seed cake used in the experiment was obtained after producing oil from camellia seeds (made in China) through a pressing method. An extract was prepared from *Camellia oleifera* seed cake using 70% ethanol, and it was confirmed that the *Camellia oleifera* seed cake extract was composed of terpine saccharides, flavonoid saccharides, sugars, and proteins. In particular, the flavonoid saccharides of *Camellia oleifera* seed cake are composed of kaempferol trisaccharides (Chemical Formulas A and B), and in this specification, it was confirmed that eight types of kaempferol 4-6 saccharide (Chemical Formulas 1-6) produced by an enzymatic reaction using two types of *Camellia oleifera* seed cake kaempferol trisaccharides as precursors had an excellent inhibitory effect on skin PDK1 activity.

### 1) Purification of Camellia oleifera seed cake kaempferol saccharide fraction

8 kg of 70% ethanol was added to 1 kg of *Camellia oleifera* seed cake from which oil was removed by low-temperature pressing, and extraction was performed at 60°C for 8 hours using a heated extractor. The extract obtained by filtering with a filter press was concentrated under reduced pressure using a rotary vacuum concentrator at 50-60°C until the solid content was about 50%, and 200 g of concentrate was obtained. This concentrate was diluted with purified water to a solid content of 10%.

This solution was loaded into a glass column filled with 2L of ion exchange resin (HP-20) and then washed with 10L of purified water to remove impurities and sugar components. Then, 10 L of 30% ethanol was sequentially added, and the eluate was concentrated under reduced pressure and freeze-dried to obtain about 20 g of camellia kaempferol saccharide fraction powder.

### 2) Purification of Camellia oleifera seed cake kaempferol trisaccharide

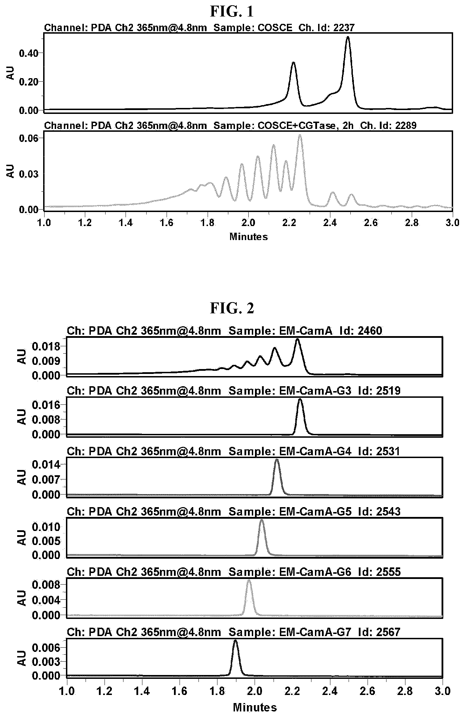

The kaempferol trisaccharide fraction derived from *Camellia oleifera* seed cake was separated using preparative HPLC. The solvent conditions used were a mixed solvent of water, acetonitrile, and methanol, and a concentration gradient of these solvents or a mixture at a constant ratio was used. As a result, components of the kaempferol trisaccharides of Chemical Formulas A and B were separated (FIG. 1).

### 3) Production and purification of kaempferol saccharides with added sugar derived from kaempferol trisaccharides

10 mg/mL of the *Camellia oleifera* seed cake extract obtained in item 2) above was dissolved using citrate phosphate buffer (pH 5, 50mM) at a concentration of 10 mg/mL water-soluble starch and 100 unit/mL of cyclodextrin glucanotransferase, and the enzyme reaction was initiated while stirring at a temperature of 50 to 70 degrees Celsius. The enzyme reaction was terminated by adding a 30% ethanol aqueous solution within 1 to 24 hours, and the supernatant was collected by centrifugation (4000g, 10 minutes) and concentrated by filtering using a 0.45 microns filter. The concentrated filtrate was adsorbed using a preparative chromatograph and a reversed-phase column (C18), and then unnecessary substances were washed with pure water. Afterwards, a wide range of peaks absorbing at UV 280 nm were collected using a concentration gradient of acetonitrile. The aliquot was concentrated and then freeze-dried to prepare a powder. (FIG. 1) For comparison, as starting materials, pure camelliaside A (Compound A), camelliaside B (Compound B), and nicotiflorin (Compound N) were subjected to enzyme reaction at 2 mg/mL and were equally aliquoted.

### 4) Pure purification of kaempferol 4-7 saccharides

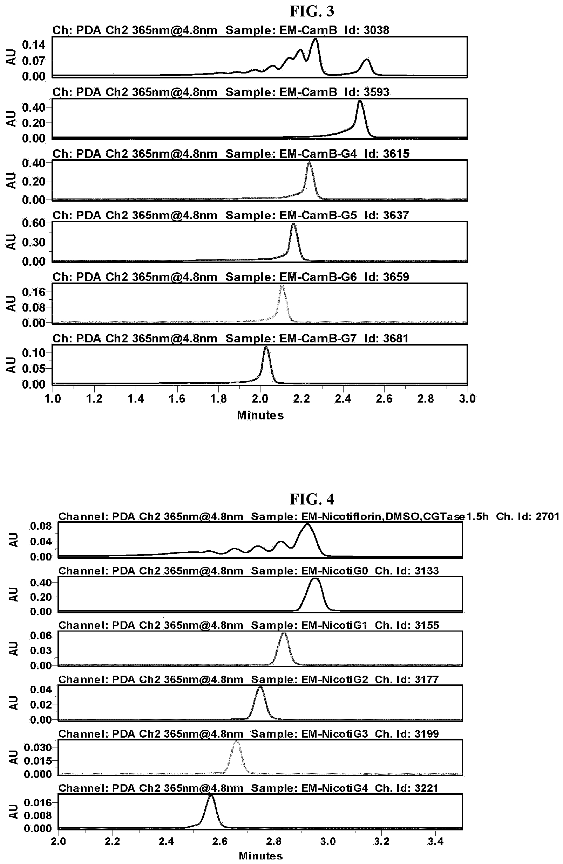

Using the pure substances camelliaside A and camelliaside B at a concentration of 2 mg/mL, a composition with an increased molecular weight was obtained through an enzymatic reaction as in the example 3) above. This was separated peak by peak using preparative chromatography and an adsorption column (C18), and four saccharides (Compounds 1-3 and A-1) derived from camelliaside A (Compound A) and four saccharides (Compounds 4-6 and B-1) derived from camelliaside B (Compound B) were obtained in purity. (FIG. 2, FIG. 3)

### 5) Identification of the structure of pure isolated substances

The structures of Compounds A, B, and N used as precursors, Compounds 1-3 and A-1, and Compounds 4-6 and B-1 obtained in step 4) above were identified using a nuclear magnetic resonance spectrometer.

### Comparative Example

In order to confirm that kaempferol trisaccharide derived from *Camellia oleifera* seed cake is meaningful as a precursor, using kaempferol disaccharide (Nicotiflorin; kaempferol rutinoside) (Compound N) as a precursor in the same method as Example 3 and Example 4, four types of saccharides (Chemicals N-1 to N-4) with increased molecular weight were produced, purified, and applied to the Test Examples (FIG. 4). In the same manner as above, the structures of Compound N, which is a precursor, and Compounds N-1 to N-4, which are reactants, were identified using a nuclear magnetic resonance analyzer.

### Test Example

### Statistics

In all of the following experiments, student *t*-test was used to determine statistical significance between samples, and a p-value of 0.05 or less was analyzed as statistically significant. (*p<0.05, **p<0.01, ***p<0.001)

### Test Example 1: PDK1 analysis

3-Phosphoinositide-dependent kinase 1 (PDK1) activity was measured using recombinant purified PDK1 enzyme and ADP-Glo + PDK1 Kinase Enzyme System (Promega, Madison, WI, USA). After dispensing the substrate, ATP, and buffer mixture, test substances (1, 3, and 10 µM) were dispensed into each well. Immediately after, PDK1 was dispensed and reacted for 60 minutes. After reaction, ADP-glo was added and incubated at room temperature for 40 minutes. Kinase detection buffer was added and reaction was performed 30 minutes and then measured with a luminescence reader (Wallac Victor2, Perkin Elmer).

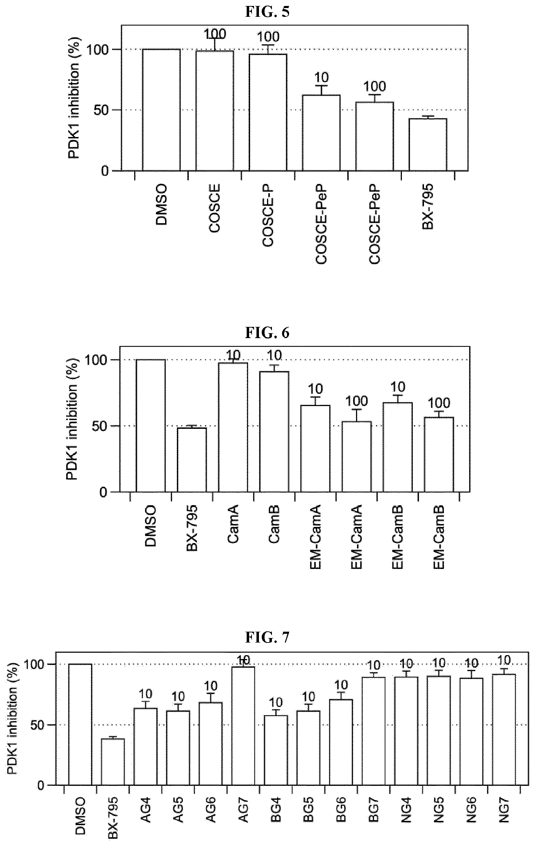

FIG. 5 is a graph showing the effect of inhibiting 3-Phosphoinositide-dependent kinase 1 (PDK1) activity on DMSO (blank), BX-795 (control), COSCE (*Camellia oleifera* seed cake extract), COSCE-P (purified product of *Camellia oleifera* seed cake extract), and COSCE-PeP (purified product of *Camellia oleifera* seed cake extract after enzyme treatment). The numerical unit above the bar is ug/mL.

Specifically, the extract contains camelliaside A and B (both trisaccharides) as its main components, and as a result of its enzymatic treatment, it contains 4, 5, 6, 7, or more saccharides including the above trisaccharides. Since it is not separated and purified, only trisaccharides are present in the extract and its purified product, and the enzyme-treated product and its purified product contain substances containing at least tetrasaccharide, including trisaccharides.

Referring to FIG. 5, it can be confirmed that the PDK activity inhibition effect of the enzyme-treated product derived from the *Camellia oleifera* seed cake extract of the present disclosure is more excellent.

Referring to FIG. 6, compared to Compound A (FIG. 6 CamA) and Compound B (FIG. 6 CamB), which are comparative examples, it can be confirmed that the PDK inhibition effect of EM-CamA containing Compounds of Chemical Formulas 1-3 and EM-CamB containing Compounds of Chemical Formulas 4-6 is more excellent.

Referring to FIG. 7, compared to Compound A-1 (FIG. 7, AG7), B-1 (FIG. 7, BG7), and Compounds N-1 to N-4 (FIG. 7, NG3-NG6), it can be confirmed that the PDK activity inhibition effect of the compound of Chemical Formulas 1-6 (AG4-AG6 and BG4-BG6) of the present disclosure is more excellent.

Specifically, it can be confirmed in FIG. 7 that the compound of Chemical Formulas 1-6 of the present disclosure noticeably inhibits PDK1 activity compared to DMSO. The degree of inhibition of PDK1 activity by the control group, NG4-NG7, did not reach the degree of inhibition by the compound (lb) of Chemical Formulas 1-6.

In addition, among kaempferol 4-7 saccharide compounds, the degree of inhibition of two types of kaempferol heptasaccharides (Compounds A-1 AG7 and B-1 BG7) was similar to that of the control compound, and this suggests that there are limitations to the size of the molecule and the degree of activity inhibition.

Moreover, the kaempferol 4-6 saccharide of the control compound (FIG. 7, Compound NG4-NG6) is similar in molecular size to the kaempferol 4-6 saccharide of the test compound, but there is a difference in activity inhibition, so this suggests that the structural specificity of the molecule affects activity inhibition.

### Embodiments

Embodiment 1: An enzyme-treated-kaempferol saccharide compound represented by any of the following Chemical Formulas 1 to 6, a stereoisomer thereof, a pharmaceutically acceptable salt thereof, a hydrate thereof, or a solvate thereof:

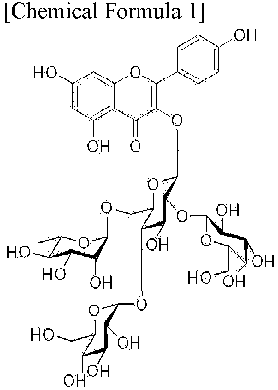

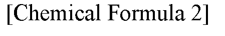

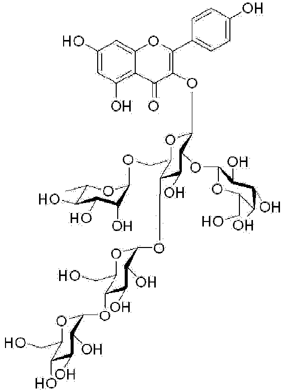

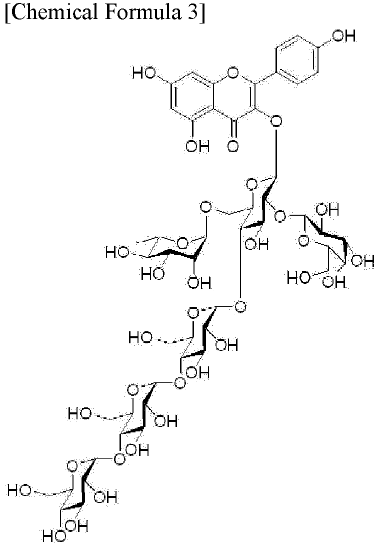

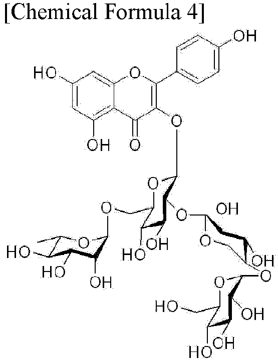

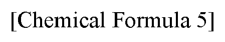

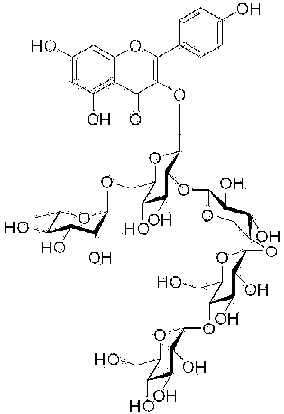

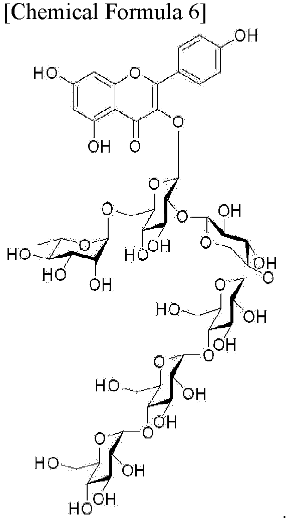

Embodiment 2: The enzyme-treated-kaempferol saccharide compound, the stereoisomer thereof, the pharmaceutically acceptable salt thereof, the hydrate thereof, or the solvate thereof according to Embodiment 1, wherein the enzyme-treated-kaempferol saccharide compound is obtained by enzymatically treating kaempferol trisaccharide.

Embodiment 3: The enzyme-treated-kaempferol saccharide compound, the stereoisomer thereof, the pharmaceutically acceptable salt thereof, the hydrate thereof, or the solvate thereof according to Embodiment 1 or 2, wherein the kaempferol trisaccharide is an extract derived from *Camellia oleifera.*

Embodiment 4: The enzyme-treated-kaempferol saccharide compound, the stereoisomer thereof, the pharmaceutically acceptable salt thereof, the hydrate thereof, or the solvate thereof according to any one of Embodiments 1 to 3, wherein the enzyme is cyclodextrin glucanotransferase.

Embodiment 5: A non-therapeutic use of a composition comprising the enzyme-treated-kaempferol saccharide compound, the stereoisomer thereof, the pharmaceutically acceptable salt thereof, the hydrate thereof, or the solvate thereof according to any one of Embodiments 1 to 4 as an active ingredient for protecting the skin.

Embodiment 6: The non-therapeutic use according to Embodiment 5, wherein the daily application amount of the active ingredient is 1 to 1000 mg/Kg.

Embodiment 7: The non-therapeutic use according to Embodiment 5 or 6, wherein the non-therapeutic use is for improving skin wrinkles.

Embodiment 8: The non-therapeutic use according to any one of Embodiments 5 to 7, wherein the non-therapeutic use is for promoting skin regeneration.

Embodiment 9: The non-therapeutic use according to any one of Embodiments 5 to 8, wherein the non-therapeutic use is for improving atopic skin.

Embodiment 10: The non-therapeutic use according to any one of Embodiments 5 to 9, wherein the non-therapeutic use is for regulating the skin keratin production cycle.

Embodiment 11: The non-therapeutic use according to any one of Embodiments 5 to 10, wherein the non-therapeutic use is for inhibiting the activity of 3-Phosphoinositide-dependent kinase 1 (PDK1).

Embodiment 12. The non-therapeutic use according to any one of Embodiments 5 to 11, wherein the non-therapeutic use is for improving skin sagging.

Embodiment 13. The non-therapeutic use according to any one of Embodiments 5 to 12, wherein the non-therapeutic use is for increasing the expression of one or more of Col7Al and Coll7Al.

Embodiment 14: The non-therapeutic use according to any one of Embodiments 5 to 13, wherein the non-therapeutic use is for improving skin barrier function.

Embodiment 15: The non-therapeutic use according to any one of Embodiments 5 to 14, wherein the non-therapeutic use is for improving skin moisturization.

Embodiment 16: The non-therapeutic use according to any one of Embodiments 5 to 15, wherein the non-therapeutic use is for increasing the expression of one or more of keratin 10 and filaggrin.

Embodiment 17: The non-therapeutic use according to any one of Embodiments 5 to 16, wherein the composition is a cosmetic composition.

Embodiment 18: The non-therapeutic use according to any one of Embodiments 5 to 17, wherein the composition is a food composition.

Embodiment 19: A method for manufacturing the enzyme-treated-kaempferol saccharide compound according to Embodiment 1, comprising:
obtaining a kaempferol saccharide compound from *Camellia oleifera* seed cake;
purifying the kaempferol saccharide compound;
enzymatically treating the purified kaempferol saccharide compound; and
purifying the enzyme-treated enzyme-treated-kaempferol saccharide compound.

Embodiment 20: The method for manufacturing the enzyme-treated-kaempferol saccharide compound according to Embodiment 19, wherein the purified kaempferol saccharide compound is a kaempferol trisaccharide compound.

Embodiment 21. The method for manufacturing the enzyme-treated-kaempferol saccharide compound according to Embodiment 19 or 20, wherein the enzyme is cyclodextrin glucanotransferase.

## Claims

1. An enzyme-treated-kaempferol saccharide compound represented by any of the following Chemical Formulas 1 to 6, a stereoisomer thereof, a pharmaceutically acceptable salt thereof, a hydrate thereof, or a solvate thereof:

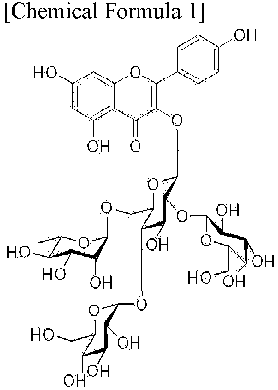

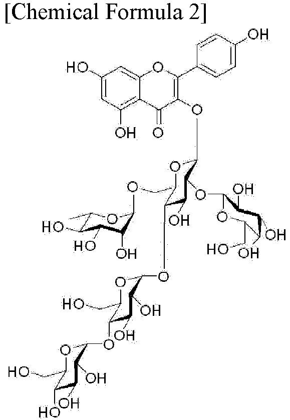

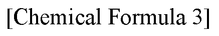

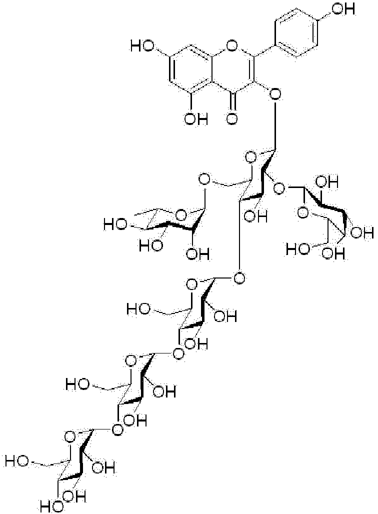

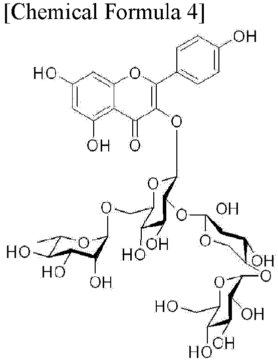

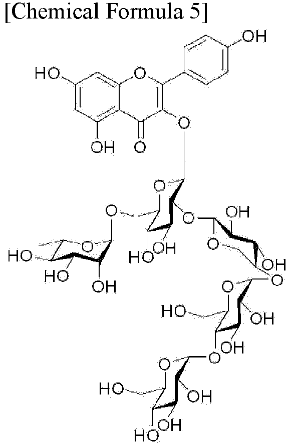

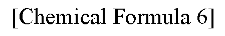

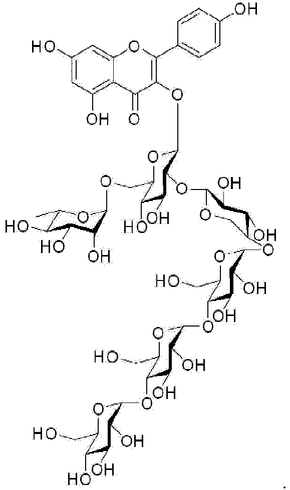

2. The enzyme-treated-kaempferol saccharide compound, the stereoisomer thereof, the pharmaceutically acceptable salt thereof, the hydrate thereof, or the solvate thereof according to claim 1, wherein the enzyme-treated-kaempferol saccharide compound is obtained by enzymatically treating kaempferol trisaccharide.

3. The enzyme-treated-kaempferol saccharide compound, the stereoisomer thereof, the pharmaceutically acceptable salt thereof, the hydrate thereof, or the solvate thereof according to claim 2, wherein the kaempferol trisaccharide is an extract derived from *Camellia oleifera.*

4. The enzyme-treated-kaempferol saccharide compound, the stereoisomer thereof, the pharmaceutically acceptable salt thereof, the hydrate thereof, or the solvate thereof according to claims 2 or 3, wherein the enzyme is cyclodextrin glucanotransferase.

5. A non-therapeutic use of a composition comprising the enzyme-treated-kaempferol saccharide compound, the stereoisomer thereof, the pharmaceutically acceptable salt thereof, the hydrate thereof, or the solvate thereof according to any one of claims 1 to 4 as an active ingredient for protecting a skin.

6. The non-therapeutic use according to claim 5, wherein a daily application amount of the active ingredient is 1 to 1000 mg/Kg.

7. The non-therapeutic use according to claims 5 or 6, wherein the non-therapeutic use is for improving skin wrinkles;
promoting skin regeneration;
improving atopic skin;
regulating the skin keratin production cycle;
inhibiting the activity of 3-Phosphoinositide-dependent kinase 1 (PDK1);
improving skin sagging;
increasing the expression of one or more of Col7A1 and Col17A1;
improving skin barrier function;
improving skin moisturization; or
increasing the expression of one or more of keratin 10 and filaggrin.

8. The non-therapeutic use according to any one of claims 5 to 7, wherein the composition is a cosmetic composition.

9. The non-therapeutic use according to any one of claims 5 to 7, wherein the composition is a food composition.

10. A method for manufacturing the enzyme-treated-kaempferol saccharide compound according to any one of claims 1 to 4, comprising:
obtaining a kaempferol saccharide compound from *Camellia oleifera* seed cake;
purifying the kaempferol saccharide compound;
enzymatically treating the purified kaempferol saccharide compound; and
purifying the enzyme-treated enzyme-treated-kaempferol saccharide compound.

11. The method for manufacturing the enzyme-treated-kaempferol saccharide compound according to claim 10,
wherein the purified kaempferol saccharide compound is a kaempferol trisaccharide compound.

12. The method for manufacturing the enzyme-treated-kaempferol saccharide compound according to claims 10 or 11,
wherein the enzyme is cyclodextrin glucanotransferase.
